# EUROPEAN PATENT APPLICATION

(11) **EP 4 148 133 A1**
(43) Date of publication of application: **15.03.2023**
(21) Application number: 21800874.6
(22) Date of filing: 07.05.2021
(51) Int. Cl.: C12N 15/62, C07K 14/75, C07K 14/78, C07K 19/00, C12N 5/071, C12N 15/12

(54) **CHIMERIC PROTEIN CONTAINING FIBRINOGEN FRAGMENT AND LAMININ FRAGMENT, AND USE THEREOF**

(30) Priority: 08.05.2020 JP 2020082877
(71) Applicant: OSAKA UNIVERSITY, Suita-shi Osaka 565-0871 (JP); Matrixome, Inc., Suita-shi, Osaka 565-0874 (JP)
(72) Inventor: SEKIGUCHI, Kiyotoshi, Suita-shi, Osaka 565-0871 (JP); TAKIZAWA, Mamoru, Suita-shi, Osaka 565-0874 (JP); TANIGUCHI, Yukimasa, Suita-shi, Osaka 565-0871 (JP)
(74) Representative: Witte, Weller & Partner Patentanwälte mbB
(86) International application number: PCT/JP2021/017594
(87) International publication number: WO 2021/225171

(57) **Abstract**

The present invention provides a gel formed of fibrin and a molecule generated by thrombin treatment of a chimeric protein that comprises a fibrinogen fragment capable of binding to fibrinogen upon thrombin treatment and a laminin fragment having integrin-binding activity, and optionally further comprises a protein having growth factor-binding activity. The gel of the present invention is suitable as a gel substrate that has properties of the basement membrane and can be used in medical applications.

## Description

### TECHNICAL FIELD

The present invention relates to a chimeric protein comprising a fibrinogen fragment and a laminin fragment and the use thereof.

### BACKGROUND ART

Cells taken from various organs are unable to proliferate and eventually die by programmed cell death, when they cannot adhere and anchor themselves to culture vessel. This property is common in cells of any organ and is referred to as "anchorage dependence of cell proliferation". Cells in the living body anchor themselves to the surrounding structure, which is called an extracellular matrix (ECM). Not less than 300 proteins have been identified as ECM components, and the molecular composition of the ECM to which cells are anchored varies with cell type. In culturing cells taken from the living body, particularly culturing stem cells, it is necessary to select a culture substrate proper for cells of interest based on the molecular composition of the ECM to which the cells are anchored in the living body.

The ECM is classified into two distinct types, a basement membrane and an interstitial matrix, based on its shape and location in the living body. The basement membrane is a sheet-like ECM of about 100 nm in thickness that forms at the boundary between the epithelium and the connective tissue and functions as an anchorage or scaffold for parenchymal cells and parenchymal stem cells in various organs to maintain their homeostasis and proliferative capacity. Therefore, in culturing cells taken from organs, particularly culturing stem cells, the culture substrate as cell culture scaffold is desirably a culture substrate that mimics the basement membrane to which the stem cells of interest are anchored in the living body.

Not less than 40 proteins have been identified as basement membrane components (Non-Patent Literature 1). Among them, collagen IV, laminin, and heparan sulfate proteoglycans such as perlecan are constitutive components of the basement membrane regardless of cell or organ type, and play an essential role in the structure and function of the basement membrane. These constitutive molecules of the basement membrane are evolutionarily well-conserved proteins and found in all metazoans studied so far.

Collagen IV is a typical collagen present in the basement membrane and self-assembles into a mesh-like structure. Laminin is an adhesion protein responsible for the scaffolding role of the basement membrane. Laminin binds to integrins on cell surfaces and mediates cell adhesion to the basement membrane. Perlecan is a typical heparan sulfate proteoglycan contained in the basement membrane. Perlecan is capable of binding to various growth factors via heparan sulfate chains attached to its domain 1 and plays a role in regulating the functions of growth factors. Heparan sulfate is an acidic glycan molecule structurally and functionally similar to heparin and is known to bind to various growth factors such as basic fibroblast growth factor (bFGF), activin A, bone morphogenetic protein (BMP), and Wnt. For the production of culture substrates that mimic the basement membrane as mentioned above, it is essential to combine laminin, which is responsible for the adhesive property of the basement membrane, and other basement membrane component molecules that are responsible for the structural or functional properties of the basement membrane. The present inventors produced a chimeric protein in which an integrin-binding domain of laminin is fused to perlecan domain 1 (Patent Literature 1) and found that this chimeric protein is useful as a substrate for inducing differentiation of pluripotent stem cells.

Crude extracts of mouse Engelbreth-Holm-Swarm (EHS) sarcoma have been widely used as a culture substrate that mimics the basement membrane. EHS sarcoma is a unique tumor that overproduces basement membrane component molecules and is known to be derived from the parietal endoderm of early mouse embryos. The extract of EHS sarcoma is commercialized under the name Matrigel (trademark) and has been used for various cell culture applications as a culture substrate that mimics the bioactivity of the basement membrane. About 60% of Matrigel is laminin-111 (α1β1γ1), and the remaining components are collagen IV, perlecan, and entactins (nidogens). Furthermore, Matrigel contains trace amounts of growth factors such as transforming growth factor (TGF)-β, insulin-like growth factor (IGF)-1, and platelet-derived growth factor (PDGF).

Matrigel gels when warmed to 22°C to 35°C. Due to this property, Matrigel is suitable for use as a three-dimensional (3D) culture substrate in which cells are embedded and cultured. Collagen gels and fibrin gels are also used for 3D cell culture. However, Matrigel has been reported more frequently to be used as a 3D culture substrate in culturing organ stem cells taken from the living body or derived from pluripotent stem cells by induced differentiation. In particular, when multiple types of cells are combined to generate a 3D organ model, called an organoid, Matrigel is most often used as a 3D culture substrate.

Matrigel is very useful as a 3D culture substrate for stem cells taken from the living body, but cells or cell clusters cultured with Matrigel have problems with medical applications. First, since Matrigel is a crude extract of mouse tumors, cells cultured with Matrigel are inevitably contaminated with proteins of mouse origin. Secondly, the chemical composition of Matrigel is known to vary from lot to lot, and standardizing its chemical composition is difficult. Thirdly, the isoform of laminin contained in Matrigel is limited to laminin-111. There are at least 12 laminin isoforms, and the laminin isoform effective for scaffolding varies with cell type. For example, laminin-111 is an effective scaffold for inducing differentiation of human iPS cells into hepatic stem cells (see Patent Literature 2), while laminin-411 is effective for inducing differentiation of human iPS cells into vascular endothelial cells (see Patent Literature 3), and laminin-332 is effective for inducing differentiation of human iPS cells into corneal epithelial cells (see Non-Patent Literature 2). However, it is technically difficult to manipulate the laminin isoform contained in Matrigel as desired.

Given this technical background, there is a pressing need for the development of 3D gel substrates usable in place of Matrigel in the fields of medical treatment and drug discovery using organ stem cells.

### CITATION LIST

### Patent Literature

Patent Literature 1: WO2012/137970
Patent Literature 2: WO2014/168157
Patent Literature 3: JP-A 2019-141086

### Non-Patent Literature

Non-Patent Literature 1:
   Manabe R, et al. Transcriptome-based systematic identification of extracellular matrix proteins. Proc. Natl. Acad. Sci. USA 105: 12849-12854, 2008
Non-Patent Literature 2:
   Shibata S, et al. Cell-type-specific adhesiveness and proliferation propensity on laminin isoforms enable purification of iPSC-derived corneal epithelium. Stem Cell Reports, 14: 663-676, 2020

### SUMMARY OF INVENTION

### TECHNICAL PROBLEM

The present invention is to provide a gel substrate that has properties of the basement membrane and can be used in medical applications.

### SOLUTION TO PROBLEM

The present invention includes the following to achieve the above-mentioned obj ect.
[1] A chimeric protein comprising a fibrinogen fragment capable of binding to fibrinogen upon thrombin treatment and a laminin fragment having integrin-binding activity.
[2] The chimeric protein according to the above [1], further comprising a protein having growth factor-binding activity.
[3] The chimeric protein according to the above [2], wherein the protein having growth factor-binding activity is a heparan sulfate proteoglycan.
[4] A gel formed of fibrin and a molecule generated by thrombin treatment of the chimeric protein according to any one of the above [1] to [3].
[5] A method for producing the gel according to the above [4], comprising the step of preparing a mixture of the chimeric protein according to any one of the above [1] to [3], fibrinogen, and thrombin.
[6] The method according to the above [5], wherein the molar ratio of the chimeric protein to the fibrinogen in the mixture is 1:5 to 1:20,000.
[7] A composition for gel preparation comprising the chimeric protein according to any one of the above [1] to [3] and fibrinogen.
[8] A kit for preparing the gel according to the above [4], the kit comprising the chimeric protein according to any one of the above [1] to [3], fibrinogen, and thrombin.
[9] A method for three-dimensional culture of cells or tissue fragments using the gel according to the above [4].
[10] A method for producing cells or organoids for transplantation medicine using the method according to the above [9].

### ADVANTAGEOUS EFFECTS OF INVENTION

The present invention provides a gel substrate that has properties of the basement membrane and can be used in medical applications. The gel substrate of the present invention can be used for 3D culture of cells and tissue fragments in place of Matrigel.

### BRIEF DESCRIPTION OF DRAWINGS

Fig. 1A shows the molecular structure of human laminin α5β1γ1E8 (LM511E8). Fig. 1B shows the molecular structure of human fibrinogen. Fig. 1C shows the molecular structure of a chimeric protein (Chimera-511) composed of human fibrinogen and integrin-binding domains of human laminin α5β1γ1.
Fig. 2 shows the results of SDS-PAGE analysis of purified LM511E8 and purified Chimera-511. The left panel shows the results of non-reducing SDS-PAGE analysis. The right panel shows the results of reducing SDS-PAGE analysis.
Fig. 3 shows the results of integrin binding assay of LM511E8 and Chimera-511.
Fig. 4 shows the results of fibrinogen binding assay of thrombin-treated LM511E8 and thrombin-treated Chimera-511.
Fig. 5 shows representative images of human iPS cells embedded in fibrin gels incorporating Chimera-511 and cultured for 7 days in culture medium with tranexamic acid (upper panels) and without tranexamic acid (lower panels).
Fig. 6 shows representative images of human iPS cells embedded in fibrin gels incorporating different concentrations of Chimera-511 and cultured for 7 days to examine the Chimera-511 concentration dependence.
Fig. 7 shows the total cell number, live cell number, dead cell number, and viability of human iPS cells collected from culture wells on day 8 of embedded culture using fibrin gels incorporating different concentrations of Chimera-511 to examine the Chimera-511 concentration dependence.
Fig. 8 shows representative images of human iPS cells embedded in fibrin gels incorporating 50 nM Chimera-511 and cultured for different periods of time until day 8 (upper panels). Lower panels show the results when human iPS cells were embedded in fibrin gels without Chimera-511 and cultured for the same periods of time (lower panels).
Fig. 9 shows representative images of human iPS cells in culture wells observed on day 8 of embedded culture using fibrin gels with Chimera-511 at a final concentration of 50 nM (A) and with LM511E8 at a final concentration of 100 nM (B) in the comparative analysis.

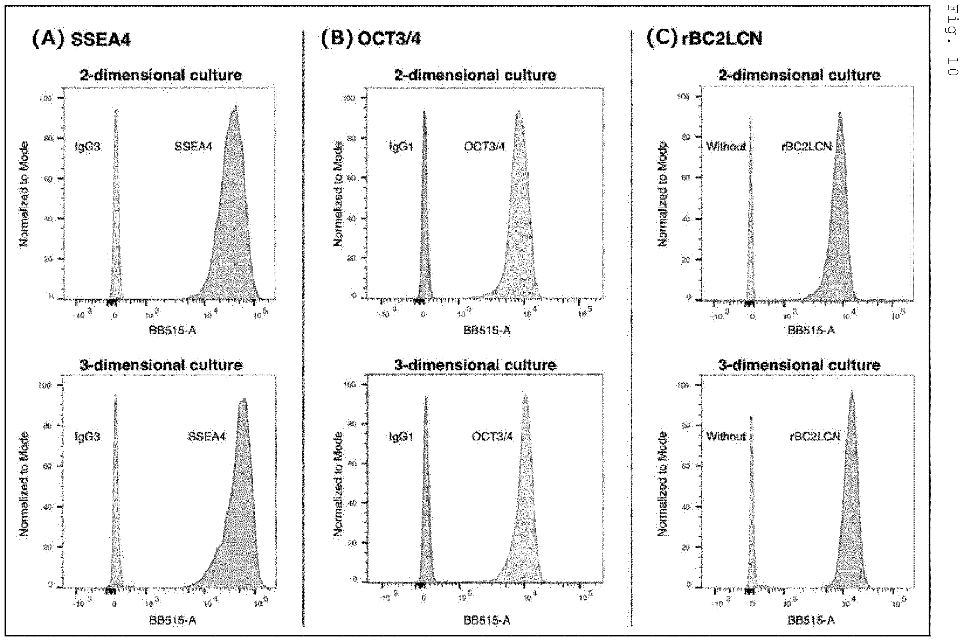

Fig. 10 shows the results of flow cytometric analysis of human iPS cells collected on day 7 of cell culture using fibrin gels incorporating Chimera-511 and stained with antibodies for undifferentiation markers in the examination of the undifferentiated nature of the human iPS cells. Fig. 10A, 10B, and 10C show the results of flow cytometric analysis of SSEA4, OCT3/4, and rBC2LCN expression, respectively. Each upper panel shows the results with human iPS cells maintained in 2D culture as a control. Each lower panel shows the results with human iPS cells embedded in the fibrin gels in 3D culture.
Fig. 11 shows the results of integrin binding assay of Chimera-5 11P.
Fig. 12 shows the results of fibrinogen binding assay of thrombin-treated Chimera-511P.
Fig. 13 shows representative images of human iPS cells in culture wells observed on day 8 of embedded culture using fibrin gels without Chimera-511P (Fibrin only, A) and with Chimera-5 11P at a final concentration of 50 nM (B) in the comparative analysis. On the left are images of the entire wells, and on the right are enlarged images of the boxed regions in the images on the left.
Fig. 14 shows the results of non-reducing SDS-PAGE analysis of chimeric proteins in culture supernatants, followed by western blotting using a His-tag antibody.
Fig. 15 shows the results of SDS-PAGE analysis of purified Chimera-111 (A), purified Chimera-221 (B), purified Chimera-332 (C), purified Chimera-411 (D), and purified Chimera-421 (E). The left panels show the results of non-reducing SDS-PAGE analysis. The right panels show the results of reducing SDS-PAGE analysis.
Fig. 16 shows the results of fibrinogen binding assay of thrombin-treated Chimera-111 (A), thrombin-treated Chimera-221 (B), thrombin-treated Chimera-332 (C), and thrombin-treated Chimera-421 (D).
Fig. 17 shows the results of integrin binding assay of thrombin-treated Chimera-111 (A), thrombin-treated Chimera-221 (B), thrombin-treated Chimera-332 (C), and thrombin-treated Chimera-421 (D) in a state of being bound to fibrinogen.
Fig. 18 shows the results of non-reducing SDS-PAGE analysis of perlecan-fused chimeric proteins in culture supernatants, followed by western blotting using a His-tag antibody.
Fig. 19 shows the results of SDS-PAGE analysis of purified Chimera-111P (A), purified Chimera-221P (B), purified Chimera-332P (C), purified Chimera-421P (D), and purified Chimera-5 11P (E). The left panels show the results of non-reducing SDS-PAGE analysis. The right panels show the results of reducing SDS-PAGE analysis.
Fig. 20 shows the results of integrin binding assay of thrombin-treated Chimera-111P (A), thrombin-treated Chimera-221P (B), thrombin-treated Chimera-332P (C), and thrombin-treated Chimera-421P (D) in a state of being bound to fibrinogen.
Fig. 21 shows the results of proactivin A binding assay of purified Chimera-111P (A), purified Chimera-221P (B), purified Chimera-332P (C), purified Chimera-421P (D), and purified Chimera-5 11P (E).
Fig. 22 shows the results of non-reducing SDS-PAGE analysis of chimeric proteins (Combination-1, Combination-2, and Combination-3) in culture supernatants, followed by western blotting using a His-tag antibody.

### DESCRIPTION OF EMBODIMENTS

### Chimeric protein

The present invention provides a chimeric protein comprising a fibrinogen fragment and a laminin fragment (hereinafter referred to as "the chimeric protein of the present invention"). The fibrinogen fragment may be any fibrinogen fragment capable of binding to fibrinogen upon thrombin treatment, and the laminin fragment may be any laminin fragment having integrin-binding activity. In other words, the chimeric protein of the present invention is capable of binding to fibrinogen upon thrombin treatment and also has integrin-binding activity. The chimeric protein may further comprise the whole or a functional domain of an additional protein. The additional protein may be a chimeric protein comprising a protein having growth factor-binding activity.

Fibrinogen is a glycoprotein that is converted to fibrin by the action of thrombin in the final step of blood coagulation. Fibrinogen is involved in blood coagulation, hemostasis, thrombus formation, wound healing, inflammation, angiogenesis, and cell-ECM interactions. Fibrinogen is a heterotrimeric molecule formed of three subunit chains termed Aα, Bβ, and γ chains associating with each other through their coiled-coil domains. Two molecules of this heterotrimer associate with each other through their N-terminal regions to form a hexamer (Aα-Bβ-γ)₂, which is the basic structure of fibrinogen. The mass numbers of the Aα, Bβ, and γ chains are 67 kDa, 56 kDa, and 47.5 kDa, respectively, and the mass number of the hexamer (Aα-Bβ-γ)₂ is 340 kDa. Fibrinogen is converted to fibrin monomers by thrombin, which cleaves fibrinogen between Arg16 and Gly 17 of the Aα chain and between Arg14 and Gly 15 of the Bβ chain to expose A and B knobs, respectively. The A and B knobs bind to the a-hole in the γ-chain C-terminal region of fibrinogen (fibrin) and the b-hole in the β-chain C-terminal region of fibrinogen (fibrin), respectively. On this basis, it will be understood that the "fibrinogen fragment capable of binding to fibrinogen upon thrombin treatment" is a fibrinogen fragment that is in the form of an Aα-Bβ-γ heterotrimer containing an A knob and a B knob at the N-terminus upon thrombin treatment.

Laminin is a major cell-adhesion molecule present in the basement membrane. Laminin is a large heterotrimeric glycoprotein with a molecular weight of 800 kDa, which consists of three subunit chains termed α, β and γ chains. The three subunit chains associate with each other through their coiled-coil domains in the C-terminal region to form a heterotrimer that is stabilized by disulfide bonds therein. There are 5 types of α chains (α1 to α5), 3 types of β chains (β1 to β3), and 3 types of γ chains (γ1 to γ3), and these chains are combined to form at least 12 different isoforms of laminins (see Table 1). The laminin fragment that constitutes the chimeric protein of the present invention may be derived from any isoform of laminin. That is, the laminin that constitutes the chimeric protein of the present invention has only to consist of one α chain selected from α1 to α5, one β chain selected from β1 to β3, and one γ chain selected from γ1 to γ3. Specifically, the 12 different isoforms shown in Table 1 and all the other possible isoforms can preferably be used.

**[Table 1]**

| α chain | Trimer composition | |
|---|---|---|
| α1 | α1β1γ1 | (laminin-111) |
| | α1β2γ1 | (laminin-121) |
| α2 | α2β1γ1 | (laminin-211) |
| | α2β2γ1 | (laminin-221) |
| | α2β1γ3 | (laminin-213) |
| α3 | α3β3γ2 | (laminin-332) |
| | α3β1γ1 | (laminin-311) |
| | α3β2γ1 | (laminin-321) |
| α4 | α4β1γ1 | (laminin-411) |
| | α4β2γ1 | (laminin-421) |
| α5 | α5β1γ1 | (laminin-511) |
| | α5β2γ1 | (laminin-521) |

The present inventors revealed that at least three C-terminal globular domains (LG1 to LG3) of the α chain and the C-terminal region of the γ chain are responsible for the integrin-binding activity of laminin. On this basis, it will be understood that the "laminin fragment having integrin-binding activity" is preferably a laminin fragment containing the C-terminal globular domains LG1 to LG3 of the α chain and the C-terminal region of the γ chain, which associate with the C-terminal region of the β chain to form a heterotrimer.

The chimeric protein of the present invention may comprise a protein having growth factor-binding activity. The protein having growth factor-binding activity is not particularly limited as long as it is capable of binding to growth factors involved in cell proliferation. Examples of the protein having growth factor-binding activity include heparan sulfate proteoglycans. Examples of the heparan sulphate proteoglycan include perlecan, agrin, collagen XVIII, syndecans 1 to 4, and glypicans 1 to 6. Examples of the growth factor-binding molecule other than heparan sulphate proteoglycans include latent TGF-β binding proteins 1 to 4. These growth factor-binding molecules may be in a full-length form or in a fragment form having growth factor-binding activity. Example of the fragment of the heparan sulfate proteoglycan having growth factor-binding activity include, for example, domain 1 of perlecan and a region containing follistatin (FS) domains 1 to 8 of agrin.

Each of the proteins that constitute the chimeric protein of the present invention (i.e., a fibrinogen fragment, a laminin fragment, and a protein having growth factor-binding activity, hereinafter referred to as "the constituent proteins of the chimeric protein") may be a protein from any organism and is not particularly limited. Each constituent protein of the chimeric protein may be a protein from mammals. Examples of the mammal include but are not limited to humans, mice, rats, cattle, and pigs. Each constituent protein of the chimeric protein can be a chimeric protein composed of proteins from different organisms, but is preferably a chimeric protein composed of proteins from the same organism. In the case where the chimeric protein is used for medical applications in humans, each constituent protein of the chimeric protein is preferably a human protein. Each constituent protein of the chimeric protein may be a native protein or a modified protein that has modification of one or more amino acid residues but retains desired biological activities of the native protein.

In the chimeric protein of the present invention, the constituent proteins of the chimeric protein may be fused together directly or via a spacer peptide(s) or linker(s). The chimeric protein of the present invention may comprise an affinity tag, such as a His tag, an HA tag, or a FLAG tag, in addition to the constituent proteins. The chimeric protein of the present invention may be labeled with a known substance for protein labeling.

The chimeric protein of the present invention can be produced as a recombinant chimeric protein by appropriate known recombinant techniques. Information regarding the nucleotide and amino acid sequences of the genes encoding fibrinogens from major mammals, the nucleotide and amino acid sequences of the genes encoding laminins from major mammals, and the nucleotide and amino acid sequences of the genes encoding proteins having growth factor-binding activity from major mammals can be obtained from known databases such as NCBI. Table 2 shows the accession numbers of the nucleotide and amino acid sequences of the genes encoding the constituent chains of human laminin. Table 3 shows the accession numbers of the nucleotide and amino acid sequences of the genes encoding the constituent chains of human fibrinogen. Table 4 shows the accession numbers of the nucleotide and amino acid sequences of the genes encoding human proteins having growth factor-binding activity.

**[Table 2]**

| | Amino acid sequence | Nucleotide sequence |
|---|---|---|
| Human laminin α1 chain | NP_005550 | NM_005559 |
| Human laminin α2 chain | NP_000417 | NM_000426 |
| Human laminin α3 chain | NP_000218 | NM_000227 |
| Human laminin α4 chain | NP_002281 | NM_002290 |
| Human laminin α5 chain | NP_005551 | NM_005560 |
| Human laminin β1 chain | NP_002282 | NM_002291 |
| Human laminin β2 chain | NP_002283 | NM_002292 |
| Human laminin β3 chain | NP_000219 | NM_000228 |
| Human laminin γ1 chain | NP_002284 | NM_002293 |
| Human laminin γ2 chain | NP_005553 | NM_005562 |
| Human laminin γ3 chain | NP_006050 | NM_006059 |

**[Table 3]**

| | Amino acid sequence | Nucleotide sequence |
|---|---|---|
| Human fibrinogen α chain | NP_068657 | NM_021871 |
| Human fibrinogen β chain | NP_005132 | NM_005141 |
| Human fibrinogen γ chain | NP_000500 | NM_000509 |

**[Table 4]**

| | Amino acid sequence | Nucleotide sequence |
|---|---|---|
| Human perlecan | NP_005520 | NM_005529 |
| Human agrin | NP_940978 | NM_198576 |
| Human collagen XVIII α1 chain | NP_085059 | NM_030582 |
| Human syndecan 1 | NP_001006947 | NM_001006946 |
| Human syndecan 2 | NP_002989 | NM_002998 |
| Human syndecan 3 | NP_055469 | NM_014654 |
| Human syndecan 4 | NP_002990 | NM_002999 |
| Human glypican 1 | NP_002072 | NM_002081 |
| Human glypican 2 | NP_689955 | NM_152742 |
| Human glypican 3 | NP_001158089 | NM_001164617 |
| Human glypican 4 | NP_001439 | NM_001448 |
| Human glypican 5 | NP_004457 | NM_004466 |
| Human glypican 6 | NP_005699 | NM_005708 |
| Human latent TGF-β binding protein 1 | NP_996826 | NM_206943 |
| Human latent TGF-β binding protein 2 | NP_000419 | NM_000428 |
| Human latent TGF-β binding protein 3 | NP_001123616 | NM_001130144 |
| Human latent TGF-β binding protein 4 | NP_001036009 | NM_001042544 |

The chimeric protein composed of a fibrinogen fragment and a laminin fragment can be produced in a form in which a heterotrimeric fibrinogen fragment containing its N-terminal region is fused to a heterotrimeric laminin fragment containing its C-terminal region. There is no limitation to the form in which a heterotrimeric fibrinogen fragment containing its N-terminal region is fused to a heterotrimeric laminin fragment containing its C-terminal region, as long as the chimeric protein is capable of binding to fibrinogen upon thrombin treatment and also has an integrin-binding activity. More specifically, the fibrinogen Aα, Bβ, and γ chains may be fused to the laminin α, β, and γ chains in any combination. In preferable embodiments, the fibrinogen Aα chain is fused to the laminin β chain, the fibrinogen Bβ chain is fused to the laminin α chain, and the fibrinogen γ chain is fused to the laminin γ chain; the fibrinogen Aα chain is fused to the laminin γ chain, the fibrinogen Bβ chain is fused to the laminin β chain, and the fibrinogen γ chain is fused to the laminin α chain; or the fibrinogen Aα chain is fused to the laminin α chain, the fibrinogen Bβ chain is fused to the laminin γ chain, and the fibrinogen γ chain is fused to the laminin β chain. The total length of each fusion chain of the chimeric protein and the ratio of the lengths of the fibrinogen and laminin chains in each fusion chain are not particularly limited, and any length and ratio can be selected as long as the chimeric protein can exert the two functions described above.

A recombinant chimeric protein composed of a fibrinogen fragment and a laminin fragment can be produced, for example, as follows. First, a DNA fragment encoding one of the fibrinogen chains is fused to a DNA fragment encoding one of the laminin chains to produce a chimeric DNA fragment. In total, three different chimeric DNA fragments are produced. Next, these three different chimeric DNA fragments are separately inserted into appropriate expression vectors to produce three different expression vectors. These three different expression vectors are co-transfected into an appropriate host cell for gene expression, and a produced trimeric protein is purified by a known method.

The chimeric protein further comprising a protein having growth factor-binding activity can be produced in a form in which the protein having growth factor-binding activity is fused to the C-terminus of at least one of the chains of the laminin fragment. For example, when a protein having growth factor-binding activity is fused to the C-terminus of the α chain of the laminin fragment, a DNA fragment encoding the protein having growth factor-binding activity is further fused to one of the above three different chimeric DNA fragments containing a DNA encoding the laminin α chain, thereby yielding a chimeric DNA fragment encoding a protein composed of one of the fibrinogen chains, the laminin α chain, and the protein having growth factor-binding activity. This chimeric DNA fragment is inserted into an appropriate expression vector and co-transfected into an appropriate host cell for gene expression together with the other two expression vectors, followed by purification of the expressed trimeric protein by a well-known method.

### Gel, method for gel production, composition for gel preparation, kit for gel preparation

The present invention provides a gel formed of fibrin and a molecule generated by thrombin treatment of the chimeric protein of the present invention described above (hereinafter referred to as "the gel of the present invention"). The present invention also provides a method for producing the gel of the present invention (hereinafter referred to as "the production method of the present invention"). The production method of the present invention comprises the step of preparing a mixture of the chimeric protein of the present invention, fibrinogen, and thrombin. As described above, the thrombin acts on the chimeric protein of the present invention and the fibrinogen, cleaving the fibrinogen Aα and Bβ chains at their N-termini to expose A and B knobs, respectively, which in turn bind to the a-hole in the C-terminal region of the fibrinogen γ chain and the b-hole in the C-terminal region of the fibrinogen β chain, respectively. This allows the formation of a fibrin gel incorporating the chimeric protein of the present invention.

The fibrinogen may be from any organism, but is preferably from the same organism as the constituent proteins of the chimeric protein of the present invention. Similarly, the thrombin may be from any organism, but is preferably from the same organism as the constituent proteins of the chimeric protein of the present invention and the fibrinogen. The chimeric protein, fibrinogen, and thrombin are all preferably from a human. In addition, the chimeric protein is preferably a chimeric protein comprising a protein having growth factor-binding activity.

The production method of the present invention can be performed by preparing a mixture of a solution of the chimeric protein of the present invention, a fibrinogen solution, and a thrombin solution. The mixture may be prepared by mixing these three separate solutions. Alternatively, the mixture may be prepared by mixing two separate solutions, e.g., a mixed solution of the chimeric protein and fibrinogen and a thrombin solution, or a mixed solution of the chimeric protein and thrombin and a fibrinogen solution. The solvent is preferably a physiological solution. For example, physiological saline, physiological buffer, or cell culture medium is suitable. The mixture of the chimeric protein, fibrinogen, and thrombin may comprise factor XIII, aprotinin, serum albumin, glycine, L-arginine hydrochloride, L-isoleucine, sodium L-glutamate, D-mannitol, sodium citrate hydrate, sodium chloride, or other substances.

The gel of the present invention preferably comprises a lysine analogue such as tranexamic acid or ε-aminocaproic acid as a fibrin stabilizer. That is, the production method of the present invention may comprise the step of preparing a mixture of the chimeric protein of the present invention, fibrinogen, thrombin, and a lysine analog (tranexamic acid or ε-aminocaproic acid). In the case of culturing cells or tissue fragments in the gel of the present invention, it is preferable to further add a cell suspension or tissue fragment suspension to the mixture.

In the production method of the present invention, the molar ratio of the chimeric protein to the fibrinogen in the mixture is preferably 1:5 to 1:20,000. The concentration of the chimeric protein in the mixture is not particularly limited and may be in the range of 0.5 nM to 1,000 nM. The concentration of the chimeric protein in the mixture may be 1 nM or more, 1.5 nM or more, 2 nM or more, 3 nM or more, 5 nM or more, 10 nM or more, 20 nM or more, 30 nM or more, 50 nM or more, 70 nM or more, or 100 nM or more, and may be 900 nM or less, 800 nM or less, 700 nM or less, 600 nM or less, 500 nM or less, or 400 nM or less.

The concentration of the fibrinogen in the mixture is not particularly limited and may be in the range of 0.1 mg/mL to 50 mg/mL. The concentration of the fibrinogen in the mixture may be 0.5 mg/mL or more, 1.0 mg/mL or more, 1.5 mg/mL or more, 2 mg/mL or more, 2.5 mg/mL or more, 3 mg/mL or more, 5 mg/mL or more, 7 mg/mL or more, 10 mg/mL or more, 15 mg/mL or more, 20 mg/mL or more, or 25 mg/mL or more, and may be 45 mg/mL or less, 40 mg/mL or less, 35 mg/mL or less, or 30 mg/mL or less. Tuning the concentration of the fibrinogen in the mixture allows the flexibility of the gel to be adjusted. That is, a higher concentration of the fibrinogen in the mixture results in a harder gel, whereas a lower concentration of the fibrinogen in the mixture results in a softer gel. The flexibility of the gel is preferably adjusted according to the intended use.

The concentration of the thrombin in the mixture is not particularly limited and can be selected according to the concentration of the fibrinogen. For example, the concentration of the thrombin in the mixture may be 0.01 NIH unit/mL to 250 NIH units/mL. The concentration of the thrombin in the mixture may be 0.05 NIH unit/mL or more, 0.1 NIH unit/mL or more, 0.5 NIH unit/mL or more, 1 NIH unit/mL or more, 2 NIH units/mL or more, 3 NIH units/mL or more, 5 NIH units/mL or more, 7 NIH units/mL or more, or 10 NIH units/mL or more, and may be 200 NIH units/mL or less, 150 NIH units/mL or less, 125 NIH units/mL or less, 100 NIH units/mL or less, 70 NIH units/mL or less, or 50 NIH units/mL or less.

The gel of the present invention may be embodied in the form of a freeze-dried gel, which is prepared by freeze-drying after gel formation. The freeze-dried gel can be swelled with a physiological solution or a cell culture medium in use. Alternatively, the freeze-dried gel may be swelled in a culture medium containing suspended cells or tissue fragments in use. The gel of the present invention may also be embodied in the form of a mixed gel with another culture gel substrate.

The present invention provides a composition for gel preparation comprising the chimeric protein of the present invention and fibrinogen (hereinafter referred to as "the composition of the present invention"). The composition of the present invention can be embodied in the form of a solution or a freeze-dried product obtained by freeze-drying a solution. The composition of the present invention may comprise factor XIII, aprotinin, tranexamic acid, ε-aminocaproic acid, serum albumin, glycine, L-arginine hydrochloride, L-isoleucine, sodium L-glutamate, D-mannitol, sodium citrate hydrate, sodium chloride, or other substances. The preferable molar ratios and preferable concentrations of the chimeric protein and the fibrinogen in the composition of the present invention are as described above in the description of the production method of the present invention.

Mixing a solution of the composition of the present invention with a thrombin solution yields the gel of the present invention. This means that the composition of the present invention can be embodied in the form of a product for preparing the gel of the present invention.

The present invention provides a kit for preparing the gel of the present invention (hereinafter referred to as "the kit of the present invention"). The kit of the present invention comprises the chimeric protein of the present invention, fibrinogen, and thrombin. More specifically, the kit can be embodied in the form of a kit comprising solutions of the chimeric protein, fibrinogen, and thrombin, or freeze-dried products of the solutions. The solutions of the chimeric protein, fibrinogen, and thrombin may be embodied in the form of three or two separate solutions. The composition of the present invention described above is suitable for use as a component of the kit of the present invention. In the case where the kit comprises freeze-dried products of the solutions of the chimeric protein, fibrinogen, and thrombin, the kit preferably comprises a solvent for dissolving these freeze-dried products. Furthermore, the kit may comprise a tube for gel preparation, an instruction manual, and other items.

### Applications of the gel of the present invention

The gel can be used for three-dimensional (3D) culture of cells or tissue fragments. That is, the present invention provides a method for 3D culture of cells or tissue fragments using the gel of the present invention described above (hereinafter referred to as the "3D culture method of the present invention"). In the 3D culture method of the present invention, any culture vessel and any culture conditions may be used as long as they allow 3D culture of cells or tissue fragments using the gel of the present invention. For example, the 3D culture method can be performed as follows: cells or tissue fragments contained in the gel are added to the wells of cell culture plates, cell culture medium is added, and culture is performed under standard culture conditions. Alternatively, cells or tissue fragments suspended in culture medium may be added to the gel in the wells of cell culture plates and then cultured under standard culture conditions.

The cells or tissue fragments used for 3D culture are not particularly limited and can be cells or tissue fragments from any organism as long as they are adaptable to 3D culture using the gel of the present invention. Preferred are cells or tissue fragments from mammals. Examples of the mammal include humans, mice, rats, cattle, and pigs. Particularly preferred are humans.

The cells can be primary cells or cell lines. The cells can be somatic cells or stem cells. The stem cells include somatic stem cells and pluripotent stem cells. The somatic stem cells include neural stem cells, mesenchymal stem cells, hematopoietic stem cells, cardiac stem cells, hepatic stem cells, and small intestinal stem cells. The pluripotent stem cells include embryonic stem cells (ES cells), induced pluripotent stem cells (iPS cells), embryonic stem cells from a cloned embryo obtained by nuclear transfer (ntES cells), spermatogenic stem cells (GS cells), embryonic germ cells (EG cells), and pluripotent cells from cultured fibroblasts or myeloid stem cells (Muse cells).

The tissue fragments may be from normal tissues or tumor tissues. In a particular embodiment, tumor cells isolated from human tumor tissue fragments are cultured using the gels of the present invention to form cancer organoids. Such cancer organoids are considered to highly reflect the characteristics of cancer cells present in human body and are useful for prediction of the sensitivity of cancer cells to cancer therapies such as chemotherapy, immunotherapy, and radiation therapy, and in addition, for anticancer drug screening and other cancer research.

In the 3D culture method of the present invention, it is preferable to select the optimal laminin isoform as a constituent protein of the chimeric protein according to the type of cells or tissue fragments to be cultured. Information on the compatibility of laminin isoforms with cell types can be easily found in the prior art literature (Masashi Yamada and Kiyotoshi Sekiguchi, Molecular Basis of Laminin-integrin Interactions, Current Topics in Membranes, 76:197-229, 2015; Lynn Yap, et al. Laminins in Cellular Differentiation, Trends in Cell Biology, 29:987-1000, 2019). It is also preferable to select the optimal softness of the gel according to the type of cells or tissue fragments.

Cells or organoids obtained using the 3D culture method of the present invention can be used for transplantation medicine. That is, the present invention provides a method for producing cells or organoids for transplantation medicine using the 3D culture method of the present invention described above. In a particular embodiment, the 3D culture method of the present invention uses the gel of the present invention produced from the chimeric protein of the present invention composed of human proteins, human fibrinogen, and human thrombin. This embodiment meets the requirements for a xeno-free environment (no xenogeneic components are contained in the culture system), so that cells or organoids obtained from human cells cultured in such an environment are suitable for human transplantation medicine.

Three-dimensionally cultured cells for transplantation medicine include, for example, cardiomyocytes, vascular endothelial cells, adipocytes, fibroblasts, myoblasts, hepatocytes, pancreatic cells, intestinal epithelial cells, alveolar epithelial cells, airway epithelial cells, kidney cells, hematopoietic cells, immune cells, nerve cells, eye tissue cells, and epidermal cells. These cells for transplantation medicine may be cells derived from stem cells cultured using the gel of the present invention.

Organoids for transplantation medicine include, for example, the kidney, liver, gastrointestinal tract (stomach, intestines, esophagus, etc.), lung and trachea, cerebrum, blood vessels, and other organs. For example, the kidney can be produced as described in Takasato et al. (Nat Protoc 11: 1681-1692, 2016) except for using the gel of the present invention instead of the 3D gel substrate described in the literature. In another example, the liver and intestines can be produced as described in Takebe et al. (Cell Rep 21: 2661-2670, 2017) and Sato et al. (Nature 459: 262-265, 2009), respectively, except for using the gel of the present invention instead of the 3D gel substrate described in the literature. In yet another example, the lung and airway can be produced as described in Yamamoto et al. (Nature Methods 14: 1097-1109, 2017) and Rock et al. (Proc Natl Acad Sci USA 106: 12771-12775, 2009), respectively, except for using the gel of the present invention instead of the 3D gel substrate described in the literature.

The gel of the present invention can be used to protect a site of transplantation in in vivo transplantation of cells, tissues, organoids, etc. For example, cells, tissues, organoids, etc. to be transplanted may be embedded in the gel of the present invention and implanted into the target site. Alternatively, the gel of the present invention may be layered over the transplanted cells, tissues, organoids, etc. at and around the transplantation site.

### EXAMPLES

Hereinafter, the present invention will be described in detail by examples, but the present invention is not limited thereto.

### Example 1: Production, purification, and activity evaluation of human fibrinogen-human laminin 511 chimeric molecule

### (1) Construction of expression vectors for human laminin 511E8 fragment

First, the multicloning site sequence of pSecTag2B was inserted into pcDNA3.4-TOPO (Thermo Fisher Scientific) according to the method of Takizawa et al. (Mamoru Takizawa et al., Sci. Adv., 2017;3:e1701497) to prepare an expression vector derived from pcDNA3.4-TOPO (hereinafter referred to as "pcDNA3.4+MCS"). Next, DNA fragments encoding human laminin α5E8 (Ala²⁵³⁴-Ala³³²⁷, with mouse Ig-κ chain V-J2-C signal peptide and 6×His tag arranged in this order from the N-terminus), human laminin β1E8 (Leu¹⁵⁶¹-Leu¹⁷⁸⁶, with mouse Ig-κ chain V-J2-C signal peptide and HA tag arranged in this order from the N-terminus), and human laminin γ1E8 (Asn¹³⁶²-Pro¹⁶⁰⁹, with mouse Ig-κ chain V-J2-C signal peptide and FLAG tag arranged in this order from the N-terminus) were excised with the restriction enzymes NheI and NotI and then separately ligated to the NheI-NotI site of the pcDNA3.4+MCS to construct expression vectors for the human laminin α5 chain fragment LMα5 (Ala²⁵³⁴-Ala³³²⁷), the human laminin β1 chain fragment LMβ1 (Leu¹⁵⁶¹-Leu¹⁷⁸⁶), and the human laminin γ1 chain fragment LMγ1 (Asn¹³⁶²-Pro¹⁶⁰⁹).

### (2) Construction of expression vectors for a chimeric protein having human fibrinogen fused to an integrin-binding domain of human laminin 511

For production of a chimeric protein having human fibrinogen fused to an integrin-binding domain of human laminin 511 (hereinafter referred to as "Chimera-511"), first, DNA fragments encoding a human fibrinogen α chain (hereinafter referred to as "FBGα (Met¹-Pro⁶⁴⁴)"), a human fibrinogen β chain (hereinafter referred to as "FBGβ (Met¹-Gln⁴⁹¹)"), and a human fibrinogen γ chain (hereinafter referred to as "FBGγ (Met¹-Val⁴³⁷)") were separately inserted into appropriate vectors to construct expression vectors for individual human fibrinogen chains. Next, expression vectors for mouse Ig-κ chain V-J2-C signal peptide/FBGβ (Gln³¹-Asn¹⁹⁴)/LMα5 (Ile²⁷¹⁶-Ala³³¹⁰) (hereinafter referred to as "Chimera-α5"), mouse Ig-κ chain V-J2-C signal peptide/FBGα (Ala²⁰-His¹⁵¹)/LMβ1 (Leu¹⁷⁶¹-Leu¹⁷⁸⁶) (hereinafter referred to as "Chimera-β1"), and mouse Ig-κ chain V-J2-C signal peptide/FBGγ (Tyr²⁷-Ser¹³²)/LMγ1 (Ile¹⁵⁷⁹-Pro¹⁶⁰⁹) (hereinafter referred to as "Chimera-y1") were constructed.

### (2-1) Construction of expression vectors for human fibrinogen

First, a human liver cDNA library was prepared from Human Liver Total RNA (Clontech, 636531) using the First Strand cDNA Synthesis Kit ReverTra Ace -α-(Toyobo, FSK-101) according to the attached protocol as follows. A mixture of 19 µL of master mix (10 µL of RNase-free H₂O, 4 µL of 5× RT Buffer, 2 µL of 10 mM dNTP Mixture, 1 µL of 10 U/µL RNase Inhibitor, 1 µL of 10 pmol/µL Oligo(dt)20, 1 µL of ReverTra Ace) and 1 µL of 1 µg/µL Human Liver Total RNA was subjected to reverse transcription reaction (incubation at 42°C for 20 min, followed by incubation at 99°C for 5 min).

Next, the human liver cDNA library was used as a template to amplify DNA fragments encoding FBGα (Met¹-Pro⁶⁴⁴), FBGβ (Met¹-Gln⁴⁹¹), and FBGγ (Met¹-Val⁴³⁷) by PCR using their respective primer sets shown below. The amplified DNA fragments were separately digested with the restriction enzymes NheI and NotI and then ligated into the NheI-NotI site of pcDNA3.4+MCS to construct expression vectors for FBGα (Met¹-Pro⁶⁴⁴), FBGβ (Met¹-Gln⁴⁹¹), and FBGγ (Met¹-VaI⁴³⁷).
(i) Primer set for FBGα (Met¹-Pro⁶⁴⁴) amplification

5’-GGGAGACCCAAGCTGGCTAGCCACCATGTTTTCCATGAGGATCGTCT
GCC-3’ (forward, SEQ ID NO: 1)

   5'-TCCTCGAGCGGCCGCCGATCTAGGGGGACAGGGAAGG-3' (reverse, SEQ ID NO: 2)
(ii) Primer set for FBGβ (Met¹-Gln⁴⁹¹) amplification

5’-TAGGGAGACCCAAGCTGGCTAGCCACCATGAAAAGGATGGTTTCTTG
GAGCTTCC-3’ (forward, SEQ ID NO: 3)

   5'-CCTCCTCGAGCGGCCGCGATCTATTGCTGTGGGAAGAAGG-3' (reverse, SEQ ID NO: 4)
(iii) Primer set for FBGγ (Met¹-Val⁴³⁷) amplification
   5'-ACCCAAGCTGGCTAGCCACCATGAGTTGGTCCTTGCACCCCCG-3' (forward, SEQ ID NO: 5)
   5'-CCCTCCTCGAGCGGCCGCGATTTAAACGTCTCCAGCCTGTTTGG-3' (reverse, SEQ ID NO: 6)

### (2-2) Construction of an expression vector for LMα5 (Ala²⁵³⁴-Ala³³¹⁰) with a C-terminal 10×His tag

The expression vector for LMα5 (Ala²⁵³⁴-Ala³³²⁷) was used as a template to amplify fragments in the 5'- and 3'-regions by PCR using their respective primer sets shown below. Both the reverse primer of the primer set (iv) and the forward primer of the primer set (v) have a sequence used for extension PCR at the 5' end.
(iv) Primer set for 5'-region fragment amplification
   5'-AGCCTGCGATGGCTCTTCCCCACCGGAGGCTCAG-3' (forward, SEQ ID NO: 7)
   5'-GTGATGGTGATGGTGATGGTGATGGTGATGGGCCTGCAGTC-3' (reverse, SEQ ID NO: 8)
(v) Primer set for 3'-region fragment amplification
   5'-AGGCCCATCACCATCACCATCACCATCACCATCACTAGATCCAGCAC-3' (forward, SEQ ID NO: 9)
   5'-GATCGAACCCTTGATGGCTGGCAACTAGAAGGCACAGTCGAGGCTG-3' (reverse, SEQ ID NO: 10)

The two DNA fragments thus obtained were ligated and amplified by extension PCR using the primer sets shown below. The amplified DNA fragment was digested with the restriction enzymes AscI and NotI and then ligated into the AscI-NotI site of the expression vector for LMα5 (Ala²⁵³⁴-Ala³³²⁷) to construct an expression vector for LMα5 (Ala²⁵³⁴-Ala³³¹⁰) with a C-terminal 10×His tag (hereinafter referred to as "LMα5 (Ala²⁵³⁴-Ala³³¹⁰)/10×His").
(vi) Primer set for ligation and amplification of LMα5 (Ala²⁵³⁴-Ala³³¹⁰)/10×His
5'-AGCCTGCGATGGCTCTTCCCCACCGGAGGCTCAG-3' (forward, SEQ ID NO: 7)

5’-GATCGAACCCTTGATGGCTGGCAACTAGAAGGCACAGTCGAGGCTG-
3’ (reverse, SEQ ID NO: 10)

### (2-3) Construction of expression vectors for Chimera-α5, Chimera-β1, and Chimera-γ1

First, the expression vectors for individual human fibrinogen chains were used as templates to amplify DNA fragments encoding FBGα (Met¹-His¹⁵¹), FBGβ (Met¹-Asn¹⁹⁴), and FBGy (Met¹-Ser¹³²) by PCR using their respective primer sets shown below. The reverse primer of each primer set has a sequence used for extension PCR at the 5' end.
(vii) Primer set for FBGα (Met¹-His¹⁵¹) amplification
   5' -AGCTCGTTTAGTGAACCGTCAGATCGCCTGGAGACGC-3' (forward, SEQ ID NO: 11) 
5’-GAACGGACTTCTCCTTCCAGTCTTGCTAAATGCTGTACTTTTTCTATG
ACTTTGCGC-3’ (reverse, SEQ ID NO: 12)
(viii) Primer set for FBGβ (Met¹-Asn¹⁹⁴) amplification
   5' -AGCTCGTTTAGTGAACCGTCAGATCGCCTGGAGACGC-3' (forward, SEQ ID NO: 11)
   5'-GCTCTCGCACGCGGCCAATGTTAGTTGGGATATTGCTATTC-3' (reverse, SEQ ID NO: 13)
(ix) Primer set for FBGγ (Met¹-Ser¹³²) amplification
   5' -AGCTCGTTTAGTGAACCGTCAGATCGCCTGGAGACGC-3' (forward, SEQ ID NO: 11)

5’-GCGAATGTCCTTCATGATCTCCTCGATACTTGAGTCATGTGTTAAAATC
GATG-3’ (reverse, SEQ ID NO: 14)

Next, the expression vectors for LMα5 (Ala²⁵³⁴-Ala³³¹⁰)/10×His, LMβ1 (Leu¹⁵⁶¹-Leu¹⁷⁸⁶), and LMγ1 (Asn¹³⁶²-Pro¹⁶⁰⁹) were used as templates to amplify DNA fragments encoding LMα5 (Ile²⁷¹⁶-Ala³³¹⁰)/10×His, LMβ1 (Leu¹⁷⁶¹-Leu¹⁷⁸⁶), and LMγ1 (Ile¹⁵⁷⁹-Pro¹⁶⁰⁹) by PCR using their respective primer sets shown below. The forward primer of each primer set has a sequence used for extension PCR at the 5' end.
(x) Primer set for LMα5 (Ile²⁷¹⁶-Ala³³¹⁰)/10×His amplification
   5'-GAATAGCAATATCCCAACTAACATTGGCCGCGTGCGAGAGC-3' (forward, SEQ ID NO: 15)
   5'-CCTTGATGGCTGGCAACTAGAAGGCACAGTCGAGGC-3' (reverse, SEQ ID NO: 16)
(xi) Primer set for LMβ1 (Leu¹⁷⁶¹-Leu¹⁷⁸⁶) amplification

5’-GCGCAAAGTCATAGAAAAAGTACAGCATTTAGCAAGACTGGAAGGA
GAAGTCCGTTC-3’ (forward, SEQ ID NO: 17)

   5'-CCTTGATGGCTGGCAACTAGAAGGCACAGTCGAGGC-3' (reverse, SEQ ID NO: 16)
(xii) Primer set for LMγ1 (Ile¹⁵⁷⁹-Pro¹⁶⁰⁹) amplification

5’-CATCGATTTTAACACATGACTCAAGTATCGAGGAGATCATGAAGGACA
TTCGC-3’ (forward, SEQ ID NO: 18)

   5'-CCTTGATGGCTGGCAACTAGAAGGCACAGTCGAGGC-3' (reverse, SEQ ID NO: 16)

The six DNA fragments thus obtained were subjected to extension PCR using the primer sets shown below to amplify three different ligated DNA fragments, which encode FBGα (Met¹-His¹⁵¹)/LMβ1 (Leu¹⁷⁶¹-Leu¹⁷⁸⁶), FBGβ (Met¹-Asn¹⁹⁴)/LMα5 (Ile²⁷¹⁶-Ala³³¹⁰)/10×His, and FBGγ (Met¹-Ser¹³²)/LMγ1 (Ile¹⁵⁷⁹-Pro¹⁶⁰⁹). The amplified DNA fragments were separately digested with the restriction enzymes NheI and NotI and then ligated to the NheI-NotI site of pcDNA3.4+MCS.
(xiii) Primer set for ligation and amplification of each chain
5'-AGCTCGTTTAGTGAACCGTCAGATCGCCTGGAGACGC-3' (forward, SEQ ID NO: 11)
5'-CCTTGATGGCTGGCAACTAGAAGGCACAGTCGAGGC-3' (reverse, SEQ ID NO: 16)

For insertion of a mouse Ig-κ chain V-J2-C signal peptide-encoding sequence, first, the expression vector for LMα5 (Ala²⁵³⁴-Ala³³²⁷) was used as a template to amplify DNA fragments encoding the mouse Ig-κ chain V-J2-C signal peptide by PCR using the primer sets shown below. The reverse primer of each primer set has a sequence used for extension PCR at the 5' end.
(xiv) Primer set for amplification of mouse Ig-κ chain V-J2-C signal peptide (for Chimera-α5)

5'-CTATATAAGCAGAGCTCGTTTAGTGAACCGTCAGATCGCCTGGAGAC
GC-3' (forward, SEQ ID NO: 19)

   5'-CATTGTCGTTGACACCTTGGTCACCAGTGGAACCTGG-3' (reverse, SEQ ID NO: 20)
(xv) Primer set for amplification of mouse Ig-κ chain V-J2-C signal peptide (for Chimera-β1)

5’-CTATATAAGCAGAGCTCGTTTAGTGAACCGTCAGATCGCCTGGAGAC
GC-3’ (forward, SEQ ID NO: 19)

   5'-CACCTTCACCACTATCTGCGTCACCAGTGGAACCTGGA-3' (reverse, SEQ ID NO: 21)
(xvi) Primer set for amplification of mouse Ig-κ chain V-J2-C signal peptide (for Chimera-γ1)

5'-CTATATAAGCAGAGCTCGTTTAGTGAACCGTCAGATCGCCTGGAGAC
GC-3' (forward, SEQ ID NO: 19)

   5'-CAGTTGTCTCTGGTAGCAACATAGTCACCAGTGGAACCTGGAACCC-3 ' (reverse, SEQ ID NO: 22)

Next, the expression vectors for FBGα (Met¹-His¹⁵¹)/LMβ1 (Leu¹⁷⁶¹-Leu¹⁷⁸⁶), FBGβ (Met¹-Asn¹⁹⁴)/LMα5 (Ile²⁷¹⁶-Ala³³¹⁰)/10×His, and FBGγ (Met¹-Ser¹³²)/LMγ1 (Ile¹⁵⁷⁹-Pro¹⁶⁰⁹) were used as templates to amplify DNA fragments encoding FBGβ (Gln³¹-Asn¹⁹⁴)/LMα5 (Ile²⁷¹⁶-Ala³³¹⁰)/10×His, FBGα (Ala²⁰-His¹⁵¹)/LMβ1 (Leu¹⁷⁶¹-Leu¹⁷⁸⁶), and FBGγ (Tyr²⁷-Ser¹³²)/LMγ1 (Ile¹⁵⁷⁹-Pro¹⁶⁰⁹) by PCR using their respective primer sets shown below. The forward primer of each primer set has a sequence used for extension PCR at the 5' end.
(xvii) Primer set for amplification of FBGβ (Gln³¹-Asn¹⁹⁴)/LMα5
   (Ile²⁷¹⁶-Ala³³¹⁰)/10×His
   5'-CCAGGTTCCACTGGTGACCAAGGTGTCAACGACAATG-3' (forward, SEQ ID NO: 23)
   5'-CCGGTAGGGATCGAACCCTTGATGGCTGGCAACTAGAAG-3' (reverse, SEQ ID NO: 24)
(xviii) Primer set for amplification of FBGα (Ala²⁰-His¹⁵¹)/LMβ1 (Leu¹⁷⁶¹-Leu¹⁷⁸⁶)
   5'-TCCAGGTTCCACTGGTGACGCAGATAGTGGTGAAGGTG-3' (forward, SEQ ID NO: 25)
   5'-CCGGTAGGGATCGAACCCTTGATGGCTGGCAACTAGAAG-3' (reverse, SEQ ID NO: 24)
(xix) Primer set for amplification of FBGγ (Tyr²⁷-Ser¹³²)/LMγ1 (Ile¹⁵⁷⁹-Pro¹⁶⁰⁹)
   5'-GGGTTCCAGGTTCCACTGGTGACTATGTTGCTACCAGAGACAACTG-3 '(forward, SEQ ID NO: 26)
   5'-CCGGTAGGGATCGAACCCTTGATGGCTGGCAACTAGAAG-3' (reverse, SEQ ID NO: 24)

The six DNA fragments amplified with the above primer sets (xiv) to (xix) were subjected to extension PCR using the primer sets shown below to amplify three different ligated DNA fragments. The amplified DNA fragments were separately digested with the restriction enzymes NheI and NotI and then ligated into the NheI-NotI site of pcDNA3.4+MCS to construct expression vectors for Chimera-α5, Chimera-β1, and Chimera-γ1.
(xx) Primer set for ligation and amplification of each chain

5'-CTATATAAGCAGAGCTCGTTTAGTGAACCGTCAGATCGCCTGGAGAC
GC-3' (forward, SEQ ID NO: 19)

5'-CCGGTAGGGATCGAACCCTTGATGGCTGGCAACTAGAAG-3' (reverse, SEQ ID NO: 24)

### (3) Expression and purification of human laminin 511E8 fragment and Chimera-511

Human laminin 511E8 (hereinafter referred to as "LM511E8") and Chimera-511 were expressed in FreeStyle 293-F cells (Thermo Fisher Scientific, hereinafter referred to as "293-F cells") transfected with the expression vectors constructed for individual subunit chains. That is, LM511E8 was expressed in 293-F cells transfected with the expression vectors for LMα5 (Ala²⁵³⁴-Ala³³²⁷), LMβ1 (Leu¹⁵⁶¹-Leu¹⁷⁸⁶), and LMγ1 (Asn¹³⁶²-Pro¹⁶⁰⁹), and Chimera-511 was expressed in 293-F cells transfected with the expression vectors for Chimera-α5, Chimera-β1, and Chimera-γ1. In each case, 400 µg each of the expression vectors for individual subunit chains were co-transfected into 1.0 × 10⁹ 293-F cells (1.0 × 10⁶ cells/mL) using the transfection reagent 293fectin (Thermo Fisher Scientific) and Opti-MEM (Thermo Fisher Scientific), and the culture medium was collected after 72 hours of culture.

The collected culture medium was centrifuged at 1,000 × g for 10 minutes, and the supernatant was further centrifuged at 15,000 × g for 30 minutes to remove remaining cells and insoluble matter. Then, 10 mL of cOmplete His-Tag Purification Resin (Roche) was added to the culture supernatant, and the protein of interest was allowed to adsorb on the resin by overnight incubation. The cOmplete His-Tag Purification Resin was collected, washed with HEPES-buffered saline adjusted to pH 8.0 (buffered saline containing 20 mM HEPES and 137 mM NaCl, hereinafter referred to as "HBS"), and subjected to elution of bound proteins with HBS (pH 8.0) containing 250 mM imidazole. The eluted fractions were identified by measurement of absorbance at 280 nm (A280).

The eluted fractions containing the protein of interest were concentrated using Amicon Ultra-15 Centrifugal Filter Units (Merck Millipore). The concentrate was subjected to gel filtration chromatography on a Superose 6 Increase 10/300GL (GE Healthcare), followed by elution with HBS (pH 7.4) at a flow rate of 0.5 mL/min. The amount of the protein of interest in the eluted fractions was determined based on absorbance at 280 nm (A280) and SDS-PAGE. The purified product obtained through gel filtration chromatography was sterilized with a 0.22-µm disk syringe filter (Merck Millipore, SLGV033RS) and stored at -80°C.

### (4) SDS-PAGE analysis of LM511E8 and Chimera-511

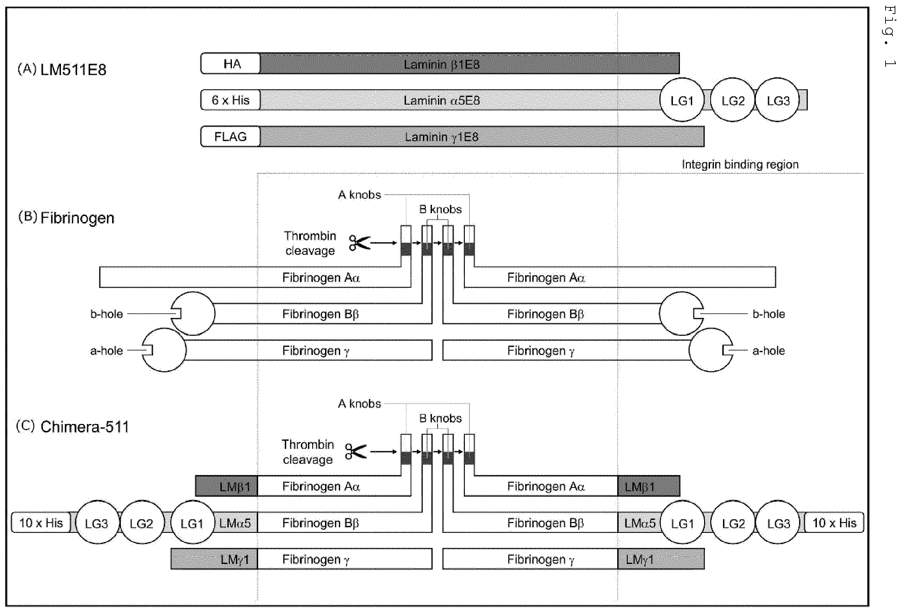

Purified LM511E8 (see Fig. 1A) and purified Chimera-511 (see Fig. 1C) were subjected to SDS-PAGE, and their electrophoretic patterns were compared. Purified LM511E8 and Chimera-511 were each applied at 1.2 µg/well on a 5-20% gradient polyacrylamide gel (ATTO, #2331830) and electrophoresed at 20 mA for 75 minutes. Electrophoresis was performed under reducing or non-reducing conditions according to the Laemmli method, using a buffer containing 25 mM Tris, 192 mM glycine, and 0.1% sodium dodecyl sulfate. For protein staining, Quick-CBB (FUJIFILM Wako Pure Chemical Corporation, #299-50101) was used.

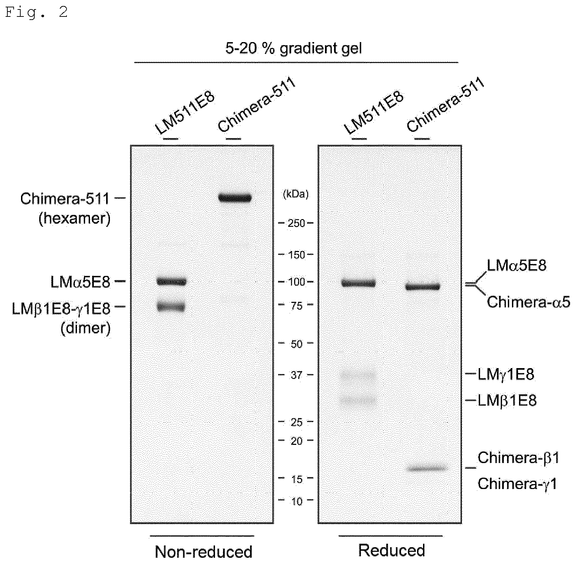

The results are shown in Fig. 2. In the non-reducing SDS-PAGE analysis, two bands were observed for LM511E8, which correspond to LMα5E8 and LMβ1E8-LMγ1E8 dimer, whereas only a single band was observed for Chimera-511, which corresponds to Chimera-511 hexamer. In the reducing SDS-PAGE analysis, three bands were observed for LM511E8, which correspond to LMα5E8, LMγ1E8, and LMβ1E8, whereas two bands were observed for Chimera-511, one band corresponding to Chimera-α5 and the other band, actually two overlapped bands corresponding to Chimera-β1 and Chimera-γ1 in the same position. These results show that the desired LM511E8 and Chimera-511 were obtained.

### (5) Integrin binding assay

### (5-1) Plate coating

LM511E8 and Chimera-511 were separately diluted to a final concentration of 10 nM in HBS (pH 7.4) and added to 96-well plates (Thermo Fisher Scientific, #442404) at 50 µL/well. The plates were incubated with gentle agitation at 4°C overnight for coating.

### (5-2) Integrin binding assay

Integrin binding assay was performed according to the method of Ido et al. (Hiroyuki Ido et al., J. Biol. Chem., 282, 11144-11154, 2007). The specific procedure was as follows. First, the LM511E8- or Chimera-511-coated 96-well plates as prepared above were washed with 200 µL/well of 20 mM Tris buffer, pH 7.4, containing 0.02% Tween-20 (FUJIFILM Wako Pure Chemical Corporation, #167-11515) and 137 mM NaCl (hereinafter referred to as "TBST") supplemented with 0.1% bovine serum albumin (BSA; Sigma-Aldrich, A7906) (hereinafter referred to as "0.1% BSA/TBST"). Next, TBST supplemented with 1% BSA was added at 200 µL/well, and the plates were incubated with agitation on a shaker (B. Braun Biotech International CERTOMAT MT) at room temperature for 1 hour for blocking. The plates were washed once with 200 µL/well of 0.1% BSA/TBST. α6β1 integrin was diluted to final concentrations of 0.001 nM, 0.003 nM, 0.01 nM, 0.03 nM, 0.1 nM, 0.3 nM, 1 nM, 3 nM, 10 nM, 30 nM, and 100 nM in 0.1% BSA/TBST containing 1 mM MnCl₂ or 10 mM EDTA. The diluted α6β1 integrin solutions were added to the plates at 50 µL/well and allowed to react with agitation using the shaker at room temperature for 3 hours.

After the reaction, the plates were washed three times with 200 µL/well of 1 mM MnCl₂/0.1% BSA/TBST or 10 mM EDTA/0.1% BSA/TBST. A 1.5 µg/mL solution of a biotin-labeled Velcro antibody (prepared as described in Takagi, J., Erickson, H. P. and Springer, T. A. (2001) Nat. Struct. Biol. 8, 412-416) in 1 mM MnCl₂/0.1% BSA/TBST was added at 50 µL/well and allowed to react with agitation using the shaker at room temperature for 30 minutes. Then, the plates were washed three times with 200 µL/well of 1 mM MnCl₂/0.1% BSA/TBST. A 0.53 µg/mL solution of streptavidin-horseradish peroxidase (Thermo Fisher Scientific, #21126) in 1 mM MnCl₂/0.1% BSA/TBST was added at 50 µL/well and allowed to react with agitation using the shaker at room temperature for 15 minutes. Then, the plates were washed three times with 200 µL/well of 1 mM MnCl₂/0.1% BSA/TBST. o-phenylenediamine (OPD; FUJIFII,M Wako Pure Chemical Corporation, #615-28-1) was dissolved at a final concentration of 0.4 mg/mL in 25 mM citric acid/50 mM Na₂HPO₄ buffer containing 0.04% H₂O₂, added at 50 µL/well, and allowed to react for 2 minutes 30 seconds. The reaction was stopped by addition of 2.5 M H₂SO₄, and the absorbance at 490 nm of the chromogenic substrate was measured with a microplate reader (Molecular Devices EMax).

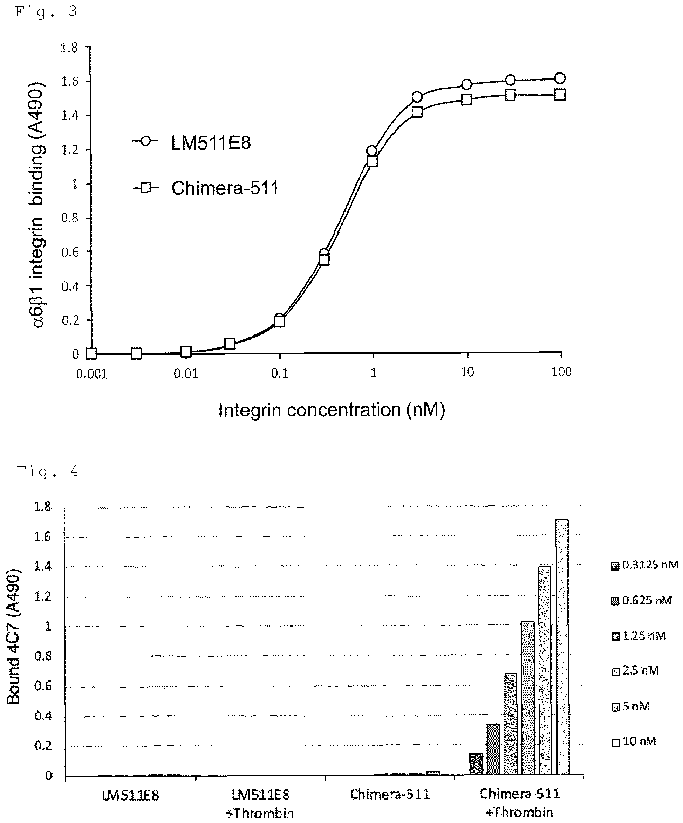

The results are shown in Fig. 3. The α6β1 integrin-binding activity of Chimera-511 was shown to be comparable to that of LM511E8. Based on the results shown in Fig. 3, the dissociation constant of α6β1 integrin for LM511E8 was 0.59 nM, and the dissociation constant of α6β1 integrin for Chimera-511 was 0.58 nM, as determined by the method of Nishiuchi et al. (Ryoko Nishiuchi et al., Matrix Biology, 25, 189-197, 2006).

### (6) Fibrinogen binding assay

### (6-1) Plate coating

Human fibrinogen was diluted to a final concentration of 100 nM in HBS (pH 7.4) and added to 96-well plates at 50 µL/well. The plates were incubated with gentle agitation at room temperature overnight for coating.

### (6-2) Binding of Chimera-511 to fibrinogen in the presence of thrombin

First, the human fibrinogen-coated 96-well plates were washed with 200 µL/well of 20 mM Tris buffer, pH 7.4, containing 1% Skim milk (Nacalai Tesque, #31149-75), 0.1% Tween-20, and 137 mM NaCl (hereinafter referred to as "1% Skim/TBST"). Next, 1% Skim/TBST was added at 200 µL/well, and the plates were incubated with agitation on a shaker at room temperature for 1 hour for blocking. The plates were washed once with TBST, and a 0.5 NIH unit/mL thrombin solution was added to the plates at 25 µL/well. LM511E8 or Chimera-511 was diluted to final concentrations of 0.3125 nM, 0.625 nM, 1.25 nM, 2.5 nM, 5 nM, and 10 nM in HBS (pH 7.4), added at 25 µL/well, and allowed to react with agitation using the shaker at room temperature for 1 hour. For controls, PBS(-) was added at 25 µL/well instead of the thrombin solution, and then the serially diluted solutions of LM511E8 or Chimera-511 were added at 25 µL/well and allowed to react with agitation using the shaker at room temperature for 1 hour in the same manner as above.

### (6-3) Measurement of bound Chimera-511

The amount of the Chimera-511 bound to fibrinogen was quantified using anti-laminin α5 chain antibody 4C7 (Merck Millipore, MAB1924). The antibody 4C7 was reported to bind to the α5E8 chain of LM511E8 (Hiroyuki Ido et al., Matrix Biology, 25, 112-117, 2006). A 1 :3000 diluted solution of the anti-laminin α5 chain antibody 4C7 in TBST was added to the plates at 50 µL/well and allowed to react with agitation using the shaker at room temperature for 1 hour. Then, the plates were washed three times with 200 µL/well of TBST. A 10 nM solution of Donkey anti-Mouse IgG/HRP (Jackson ImmunoResearch Laboratories, #715-035-150) in TBST was added at 50 µL/well and allowed to react with agitation using the shaker at room temperature for 1 hour. Then, the plates were washed three times with 200 µL/well of TBST. o-phenylenediamine was dissolved at a final concentration of 0.4 mg/mL in 25 mM citric acid/50 mM Na₂HPO₄ buffer containing 0.04% H₂O₂, added at 50 µL/well, and allowed to react for 2 minutes. The reaction was stopped by addition of 2.5 M H₂SO₄, and the absorbance at 490 nm of the chromogenic substrate was measured with a microplate reader.

The results are shown in Fig. 4. The amount of the Chimera-511 bound to fibrinogen increased in a concentration-dependent manner in the presence of thrombin, but Chimera-511 did not bind to fibrinogen in the absence of thrombin. In contrast, LM511E8 did not bind to fibrinogen with or without thrombin. The results indicate that thrombin cleaved the N-terminal regions of the Chimera-aS and Chimera-β1 chains in Chimera-511, thereby exposing the A and B knobs (see Fig. 1), which in turn bound to the a- and b-holes of the fibrinogen on the plates, respectively.

### Example 2: Examination of the effect of tranexamic acid supplementation on cell culture using a fibrin gel incorporating Chimera-511

### (1) Maintenance culture of human iPS cells

The human iPS cells (hereinafter referred to as "hiPS cells") used were human iPS cell line 201B7, which was purchased from RIKEN BioResource Research Center. The hiPS cells were maintained on 6-well plates (Corning, #353046) according to a partially modified version of the protocol of Nakagawa et al. (Nakagawa et al., Sci. Rep. 4:3594, doi:10.1038/srep03594, 2014). A cell detachment solution (TrypLE Select (Thermo Fisher Scientific) diluted 1:1 with 0.5 mM EDTA/PBS(-)) was added to the hiPS cells on the plates. After incubation at 37°C for 5 minutes, the hiPS cells were detached, and a hiPS cell suspension was prepared. More specifically, after the incubation, the cell detachment solution was aspirated off, and the hiPS cells were washed with PBS(-). Then, StemFit AK02N medium (Ajinomoto) containing 10 µM ROCK inhibitor (Y-27632, FUJIFII,M Wako Pure Chemical Corporation) was added at 1 mL/well, and the hiPS cells were harvested using a cell scraper (Sumitomo Bakelite). The hiPS cells were dissociated into single cells by repeated pipetting, and 10 µL of the cell suspension was mixed with 10 µL of 0.4% trypan blue (Thermo Fisher Scientific, T10282). The cell number was measured using a Countess automated cell counter (Thermo Fisher Scientific).

On the day before the start of culture, a 22 nM LM511E8 coating solution was added to 6-well culture plates at 1.5 mL/well, and the plates were incubated at 4°C overnight for coating. On the first day of culture, a 1 mg/mL solution of recombinant human serum albumin (Novozymes) in PBS(-) was added at 1.5 mL/well to prevent drying-caused inactivation of the LM511E8 on the plates. The solution remaining in each well was aspirated off, the 201B7 hiPS cells dissociated as single cells were seeded at 1.0 × 10⁴ cells/well, and culture was started under 37°C/0.5% CO₂ conditions. Culture media were changed with StemFit AK02N on the day after the start of culture, day 4, day 5, and day 6 of culture. The hiPS cells on day 7 of culture were used for cell culture using fibrin gels.

### (2) Embedded culture of hiPS cells using fibrin gels

### (2-1) Preparation of lower layer gels

Human fibrinogen (Enzyme Research Laboratories, FIB 3) was diluted to 5 mg/mL in StemFit AK02N to prepare a 2× fibrinogen solution. Thrombin (Sigma-Aldrich, T4393) was diluted to 1 NIH unit/mL in StemFit AK02N to prepare a 2× thrombin solution. To a 1.7 mL tube containing 250 µL of the 2× fibrinogen solution, 250 µL of the 2× thrombin solution was added. After mixing by pipetting and inversion, 250 µL aliquots of the mixture were added to 24-well culture plates (BD Biosciences) and incubated at 37°C for 10 minutes for gelation. Then, 10 µM Y-27632/StemFit AK02N was added at 1 mL/well onto the fibrin gel in the wells for the tranexamic acid-free medium group, while 10 µM Y-27632/1 mM tranexamic acid/StemFit AK02N was added at 1 mL/well onto the fibrin gel in the wells for the tranexamic acid-containing medium group. The plates were incubated under 37°C/0.5% CO₂ conditions.

### (2-2) Preparation of upper layer gels and maintenance culture of hiPS cells

The hiPS cells on day 7 of culture were detached, dissociated into single cells, and counted according to the same procedure as described above. A 2× cell suspension containing 4 × 10⁴ cells/mL cells, 1 NIH unit/mL thrombin, and 100 nM Chimera-511 was prepared. To a 1.7 mL tube containing 250 µL of the 2× fibrinogen solution, 250 µL of the 2× cell suspension was added. After mixing by pipetting and inversion, 250 µL aliquots of the mixture were added to each well containing the lower gel and incubated at 37°C for 10 minutes for gelation. Then, 10 µM Y-27632/StemFit AK02N was added at 1 mL/well onto the fibrin gel in the wells for the tranexamic acid-free medium group, while 10 µM Y-27632/1 mM tranexamic acid/StemFit AK02N was added at 1 mL/well onto the fibrin gel in the wells for the tranexamic acid-containing medium group. Embedded culture was started under 37°C/0.5% CO₂ conditions. Culture media were changed with 1 mM tranexamic acid/StemFit AK02N on the day after the start of culture, day 4, day 5, and day 6 of culture.

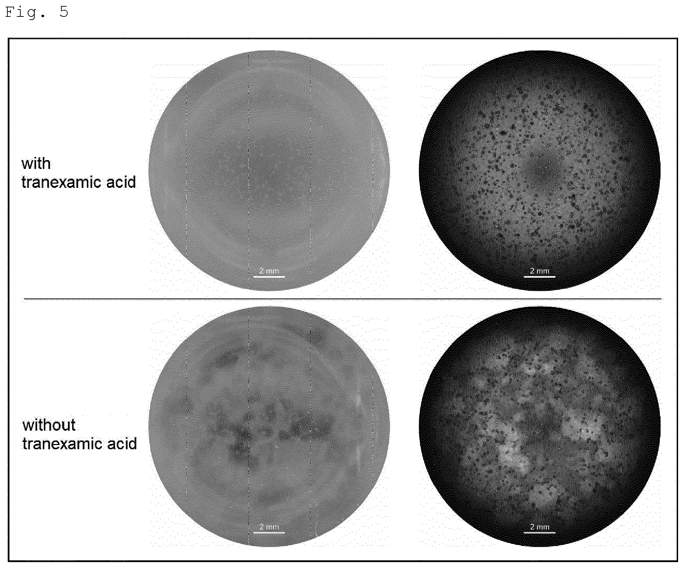

The results on day 7 of culture are shown in Fig. 5. The upper panels show representative images of the wells in the tranexamic acid-containing medium group. The lower panels show representative images of the wells in the tranexamic acid-free medium group. On the left are bright-field images, and on the right are phase contrast images. In the tranexamic acid-free medium group, gel dissolution became apparent from day 4 of embedded culture, and occasionally, some cells together with part of the gel came off at medium change. In contrast, in the tranexamic acid-containing medium group, no gel dissolution was observed until the end of the observation period, and the cells were grown to form clusters having an almost true sphere shape. The "no gel dissolution" observed in the tranexamic acid-containing medium group can be explained by the anti-plasmin effect of tranexamic acid.

### Example 3: Examination of Chimera-511 concentration dependence in cell culture using fibrin gels incorporating Chimera-511

Embedded culture of hiPS cells was performed according to the same procedure as described in Example 2, except that Chimera-511 was added to the 2× cell suspension such that the final concentration of Chimera-511 was 0.15 nM, 0.5 nM, 1.5 nM, 5 nM, 15 nM, 50 nM, or 150 nM. For a negative control, embedded culture of hiPS cells was performed using a fibrin gel without Chimera-511. Cell clusters were photographed using an all-in-one fluorescence microscope (Keyence, BZ-X710) on the day after cell embedding, day 4, day 5, day 6, and day 7 of culture to examine Chimera-511 concentration dependence in cell cluster formation.

On day 8 of culture, the cells were collected and counted as follows. The cell culture medium was aspirated off, and the cells were washed with PBS(-). A fibrin gel dissolution solution [PBS(-) containing 2.5 mg/mL trypsin (Thermo Fisher Scientific, #15090046), 5 mM EDTA, and 10 µM Y-27632] was added at 1 mL/well, and the plates were incubated on a shaker at 37°C for 30 min for fibrin gel digestion. The hiPS cells were dissociated into single cells by repeated pipetting, and the supernatant was removed by centrifugation (300 × g) for 5 minutes. The hiPS cells were resuspended in a fibrin gel dissolution solution. Then, 10 µL of the cell suspension was mixed with 10 µL of 0.4% trypan blue (Thermo Fisher Scientific, T10282), and the cell number was measured using a Countess automated cell counter (Thermo Fisher Scientific). The total cell number and trypan blue-stained cell number were measured, and the viability was calculated.

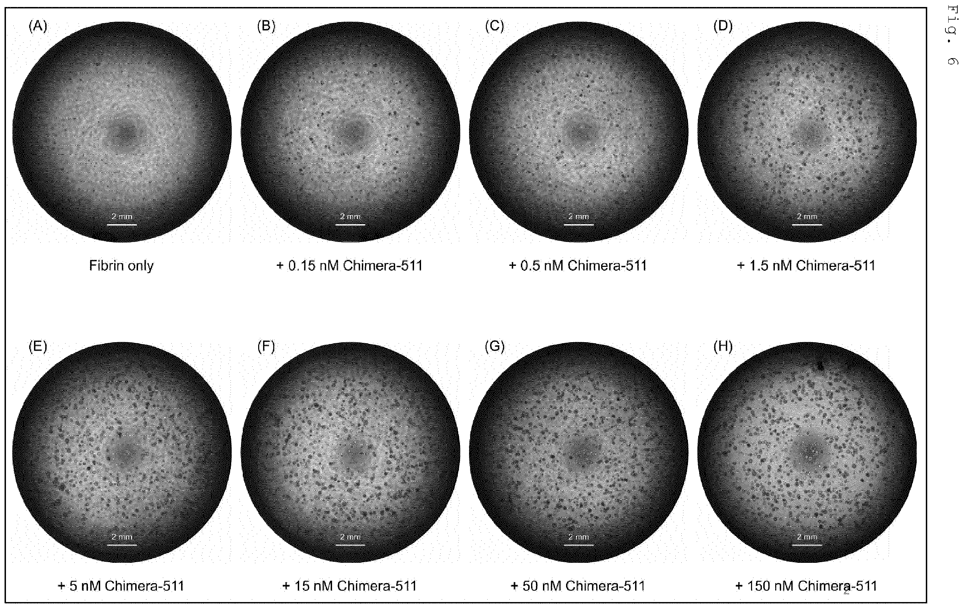

The results of cell culture on day 7 are shown in Fig. 6. The number of cell clusters increased in a Chimera-511 concentration-dependent manner.

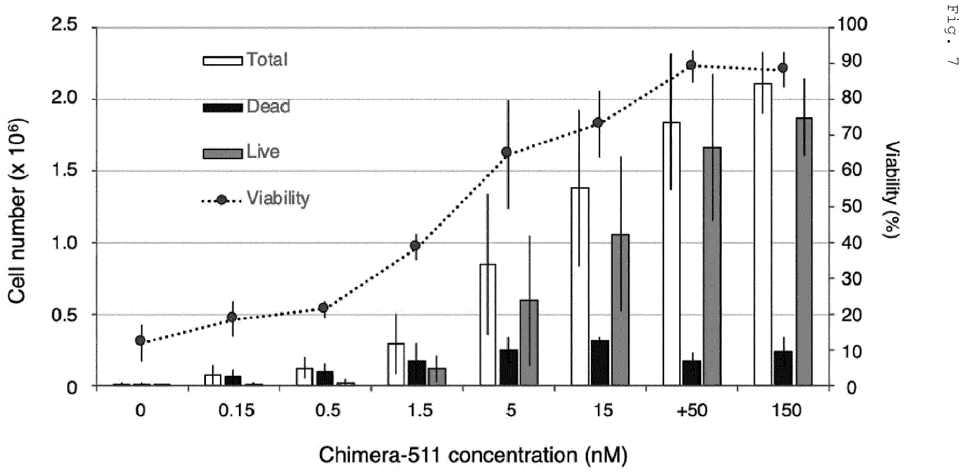

The results on cell number and viability on day 8 of culture are shown in Fig. 7. The data in Fig. 7 are presented as means and standard deviations of three independent experiments. The cell number and viability increased in a Chimera-511 concentration-dependent manner.

### Example 4: Examination of time course changes in cell culture using a fibrin gel incorporating Chimera-511

Embedded culture of hiPS cells was performed according to the same procedure as described in Example 2 with the final Chimera-511 concentration of 50 nM. For a negative control, embedded culture of hiPS cells was performed using a fibrin gel without Chimera-511. Cell clusters were photographed using an all-in-one fluorescence microscope (Keyence, BZ-X710) on the day after cell embedding (day 1 of culture), day 4, day 6, and day 8 of culture to examine time course changes.

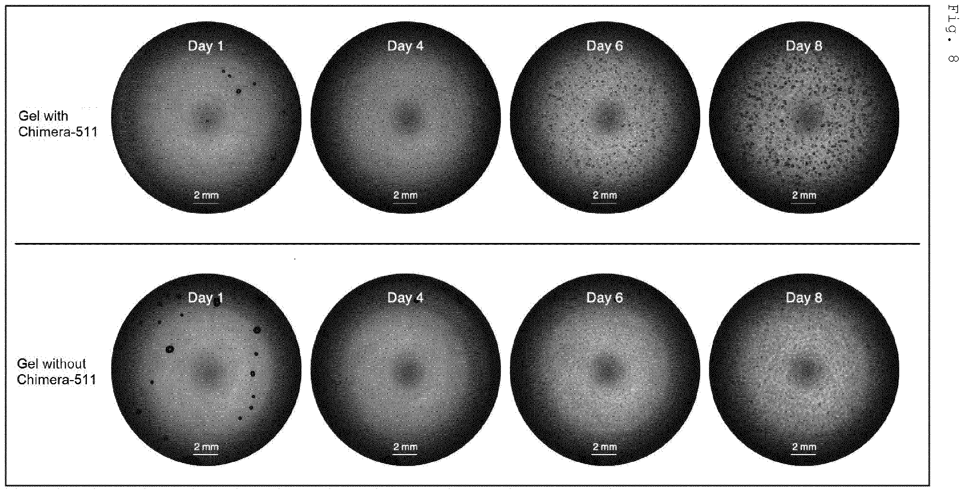

The results are shown in Fig. 8. The upper panels show the images of hiPS cells cultured using the fibrin gel incorporating Chimera-511. The lower panels show the images of hiPS cells cultured using the fibrin gel without Chimera-511. Cell clusters grew and became larger in a time-dependent manner on the fibrin gel incorporating Chimera-511. In contrast, no cell clusters were grown on the fibrin gel without Chimera-511, even on day 8 of culture.

### Example 5: Comparison of the effect of Chimera-511 or LM511E8 supplementation on cell culture using a fibrin gel

Embedded culture of hiPS cells was performed according to the same procedure as described in Example 2 using a fibrin gel incorporating Chimera-511 at a final concentration of 50 nM. Similarly, embedded culture of hiPS cells was performed using a fibrin gel incorporating LM511E8 at a final concentration of 100 nM in place of Chimera-511. Cell clusters were photographed using an all-in-one fluorescence microscope (Keyence, BZ-X710) on day 8 of culture, and cell cluster growth was compared.

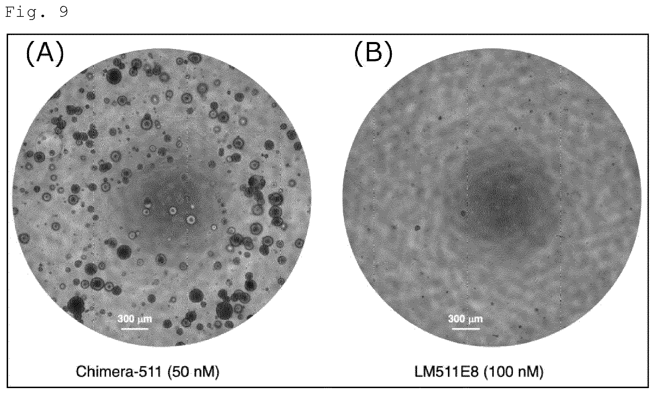

The results are shown in Fig. 9. Fig. 9A shows the results of cell culture using the fibrin gel with Chimera-511. Fig. 9B shows the results of cell culture using the fibrin gel with LM511E8. A large number of cell clusters of grown hiPS cells were observed when the fibrin gel with Chimera-511 was used, but such cell clusters of hiPS cells were hardly observed when the fibrin gel with LM511E8 was used. These results indicate that the inclusion of LM511E8 only having integrin-binding activity in fibrin gels is not sufficient for cell growth and that the inclusion of fibrin gel-bound LM511 fragments having integrin-binding activity is necessary for cell growth.

### Example 6: Examination of the undifferentiated nature of hiPS cells cultured using a fibrin gel incorporating Chimera-511

Embedded culture of hiPS cells was performed according to the same procedure as described in Example 2 with the final Chimera-511 concentration of 50 nM. On day 7 of culture, the cell culture medium was aspirated off, and the cells were washed with PBS(-). A fibrin gel dissolution solution [PBS(-) containing 2.5 mg/mL trypsin, 5 mM EDTA, and 10 µM Y-27632] was added at 1 mL/well. The plates were incubated with agitation on a shaker at 37°C for 30 minutes for fibrin gel digestion. The hiPS cells were dissociated into single cells by repeated pipetting, and the supernatant was removed by centrifugation (300 × g) for 5 minutes. The hiPS cells were resuspended in 10 µM Y-27632/PBS(-) and filtered through a 40-µm cell strainer (Corning, #352340). The hiPS cells were washed once with 10 µM Y-27632/PBS(-), resuspended in 10 µM Y-27632/PBS(-), and counted using a Countess automated cell counter.

The hiPS cells were suspended in a 3.7% paraformaldehyde solution in PBS(-) and fixed at room temperature for 10 minutes. The hiPS cells were washed twice with PBS(-), suspended in PBS(-) containing 1.5% fetal bovine serum (FBS), and incubated at 4°C overnight. To this cell suspension, the following antibodies, isotype controls or recombinant protein were added, and the mixture was allowed to stand on ice for 1 hour.
- FITC Mouse anti-SSEA4 (BD, #560126)
- FITC Mouse IgG3 control (BD, #555588)
- Alexa Fluor 488 Mouse anti-Oct3/4 (BD, #555588)
- Alexa Fluor 488 Mouse IgG1 Isotype control (BD, #557782)
- rBC2LCN-FITC (FUJIFILM Wako Pure Chemical Corporation, #180-01192)

For staining with Alexa Fluor 488 Mouse anti-Oct3/4 or Alexa Fluor 488 Mouse IgG1 Isotype control, the cell suspension was subjected to permeabilization with 0.2% NP-40 in parallel with the antibody reaction. For a negative control for rBC2LCN-FITC, a cell suspension was prepared without the addition of rBC2LCN-FITC. After the reaction, the antibody reaction solutions were removed. The hiPS cells were washed once with PBS(-) containing 1.5% FBS and resuspended in D-PBS. The cell suspensions were kept on ice. The cells were analyzed for undifferentiation marker expression with a BD FACSCelesta flow cytometer.
hiPS cells maintained in two-dimensional (2D) culture on 6-well plates (Corning, #353046) were used as a control. A cell detachment solution was added to the hiPS cells on day 7 of maintenance culture, and incubation was performed at 37°C for 5 minutes. The cell detachment solution was aspirated off, and the hiPS cells were washed with PBS(-). Then, StemFit AK02N medium containing 10 µM Y-27632 was added at 1 mL/well, and the hiPS cells were harvested using a cell scraper. The hiPS cells were dissociated into single cells by repeated pipetting, and 10 µL of the cell suspension was mixed with 10 µL of 0.4% trypan blue. The cell number was measured using a Countess automated cell counter. The harvested hiPS cells were washed twice with 10 µM Y-27632/PBS(-), suspended in a 3.7% paraformaldehyde solution in PBS(-), and fixed at room temperature for 10 minutes. Subsequently, antibody reaction and undifferentiation marker expression analysis were performed according to the same procedure as described above for the hiPS cells embedded in fibrin gels in 3D culture.

The results are shown in Fig. 10. Fig. 10A, 10B, and 10C show the results of flow cytometric analysis of SSEA4, OCT3/4, and rBC2LCN expression, respectively. Each upper panel shows the results with hiPS cells maintained in 2D culture. Each lower panel shows the results with hiPS cells embedded in fibrin gels in 3D culture. The results show that the hiPS cells embedded in fibrin gels in 3D culture were maintained in an undifferentiated state similarly to the hiPS cells maintained in 2D-culture.

### Example 7: Production, purification, and activity evaluation of perlecan-fused Chimera-511

### (1) Construction of an expression vector for human perlecan domain 1-fused Chimera-α5

Perlecan is a major heparan sulfate proteoglycan of the basement membrane. Heparan sulfate chains of perlecan have growth factor-binding activity and are attached to domain 1 of perlecan (perlecan D 1). A chimeric protein having human perlecan D 1 fused to the C-terminus of Chimera-α5 (hereinafter referred to as "Chimera-α5(+P)") was produced as Chimera-511 having growth factor-binding activity in the following manner. First, an expression vector for a chimeric protein composed of human perlecan D1 and LMα5E8 (hereinafter referred to as "LMα5E8(+P)") was constructed according to the procedure described in WO2014/199754A1, and then digested with the restriction enzymes ClaI and NotI to excise a DNA fragment encoding the C-terminal region of LMα5E8(+P), which contained perlecan D1. Next, this DNA fragment was ligated to the ClaI-NotI site of the expression vector for Chimera-aS to construct an expression vector for Chimera-α5(+P).

### (2) Expression and purification of perlecan-fused Chimera-511 (hereinafter referred to as "Chimera-511P")

Chimera-5 11P was expressed in 293-F cells transfected with the expression vectors constructed for individual subunit chains. That is, Chimera-5 11P was expressed in 293-F cells transfected with the expression vectors for Chimera-α5(+P), Chimera-β1, and Chimera-γ1. Specifically, 400 µg each of the expression vectors for individual subunit chains were co-transfected into 1.0 × 10⁹ 293-F cells (1.0 × 10⁶ cells/mL) using the transfection reagent 293fectin and Opti-MEM, and the culture medium was collected after 72 hours of culture. The collected culture medium was centrifuged at 1,000 × g for 10 minutes, and the supernatant was further centrifuged at 15,000 × g for 30 minutes to remove remaining cells and insoluble matter. Then, 10 mL of cOmplete His-Tag Purification Resin (Roche) was added to the culture supernatant, and the protein of interest was allowed to adsorb on the resin by overnight incubation. The cOmplete His-Tag Purification Resin was collected, washed with HBS (pH 8.0), and subjected to elution of bound proteins with HBS containing 250 mM imidazole (pH 8.0). The eluted fractions were identified by measurement of absorbance at 280 nm (A280).

The eluted fractions containing the protein of interest were concentrated using Amicon Ultra-15 Centrifugal Filter Units (Merck Millipore). The concentrate was subjected to gel filtration chromatography on a Superose 6 Increase 10/300GL (GE Healthcare), followed by elution with HBS (pH 7.4) at a flow rate of 0.5 mL/min. The amount of the protein of interest in the eluted fractions was determined based on absorbance at 280 nm (A280) and SDS-PAGE. The purified product obtained through gel filtration chromatography was sterilized with a 0.22-µm disk syringe filter (Merck Millipore, SLGV033RS) and stored at -80°C.

### (3) Integrin binding assay

The α6β1 integrin-binding activity of Chimera-511P was measured using the same method as described in Example 1 (5).

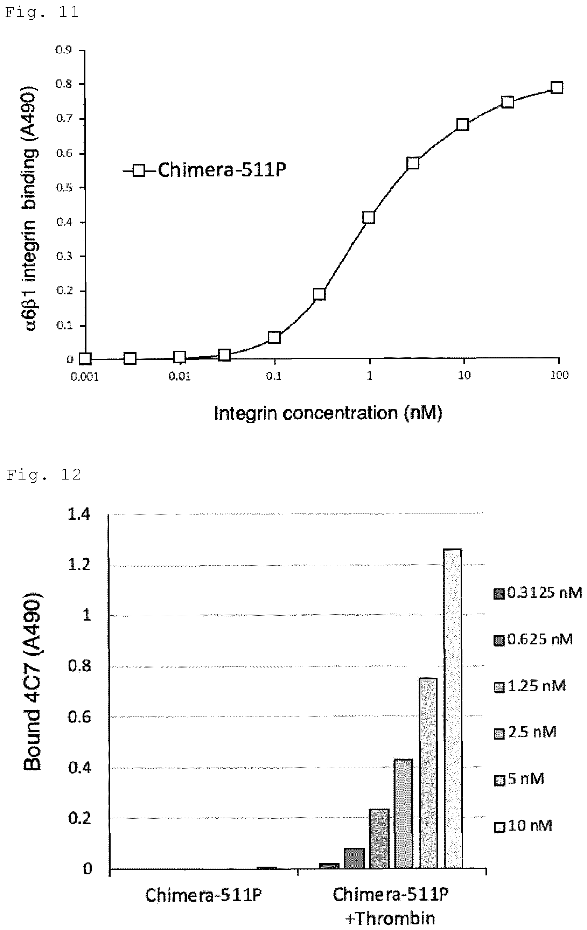

The results of the integrin binding assay are shown in Fig. 11. The amount of the α6β1 integrin bound to Chimera-5 11P increased in a concentration-dependent manner with an apparent dissociation constant of 0.90 nM. These results indicate that Chimera-5 11P retains an α6β1 integrin-binding activity almost comparable to those of Chimera-511 and LM511E8.

### (4) Fibrinogen binding assay

### (4-1) Plate coating

Human fibrinogen was diluted to a final concentration of 100 nM in HBS (pH 7.4) and added to 96-well plates at 50 µL/well. The plates were incubated with gentle agitation at room temperature overnight for coating.

### (4-2) Binding of Chimera-5 11P to fibrinogen in the presence of thrombin

The human fibrinogen-coated 96-well plates were washed with 200 µL/well of 1% Skim/TBST. Next, 1% Skim/TBST was added at 200 µL/well, and the plates were incubated with agitation on a shaker at room temperature for 1 hour for blocking. The plates were washed once with TBST, and a 0.5 NIH unit/mL thrombin solution was added to the plates at 25 µL/well. Chimera-5 11P was diluted to final concentrations of 0.3125 nM, 0.625 nM, 1.25 nM, 2.5 nM, 5 nM, and 10 nM in HBS (pH 7.4), added at 25 µL/well, and allowed to react with agitation using the shaker at room temperature for 1 hour. For controls, PBS(-) was added at 25 µL/well instead of the thrombin solution, and then the serially diluted solutions of Chimera-5 11P were added at 25 µL/well and allowed to react with agitation using the shaker at room temperature for 1 hour in the same manner as above.

### (4-3) Measurement of amount of bound Chimera-5 11P

The amount of the Chimera-511P bound to fibrinogen was quantified using anti-laminin α5 chain antibody 4C7 (Merck Millipore, MAB1924). A 1:3000 diluted solution of the anti-laminin α5 chain antibody 4C7 in TBST was added to the plates at 50 µL/well and allowed to react with agitation using the shaker at room temperature for 1 hour. Then, the plates were washed three times with 200 µL/well of TBST. A 10 nM solution of Donkey anti-Mouse IgG/HRP (Jackson ImmunoResearch Laboratories, #715-035-150) in TBST was added at 50 µL/well and allowed to react with agitation using the shaker at room temperature for 1 hour. Then, the plates were washed three times with 200 µL/wellof TBST. o-phenylenediamine was dissolved at a final concentration of 0.4 mg/mL in 25 mM citric acid/50 mM Na₂HPO₄ buffer containing 0.04% H₂O₂, added at 50 µL/well, and allowed to react for 2 minutes. The reaction was stopped by addition of 2.5 M H₂SO₄, and the absorbance at 490 nm of the chromogenic substrate was measured with a microplate reader.

The results are shown in Fig. 12. The amount of the Chimera-511P bound to fibrinogen increased in a concentration-dependent manner in the presence of thrombin, but Chimera-5 11P did not bind to fibrinogen in the absence of thrombin. The results indicate that, as with Chimera-511, thrombin cleaved the N-terminal regions of the Chimera-α5(+P) and Chimera-β1 chains in Chimera-511P, thereby exposing the A and B knobs, which in turn bound to the a- and b-holes of the fibrinogen on the plates, respectively.

### Example 8: Cell culture using a fibrin gel incorporating Chimera-5 11P

Embedded culture of hiPS cells was performed according to the same procedure as described in Example 2 with the final Chimera-5 11P concentration of 50 nM. For a negative control, embedded culture of hiPS cells was performed using a fibrin gel without Chimera-511P.

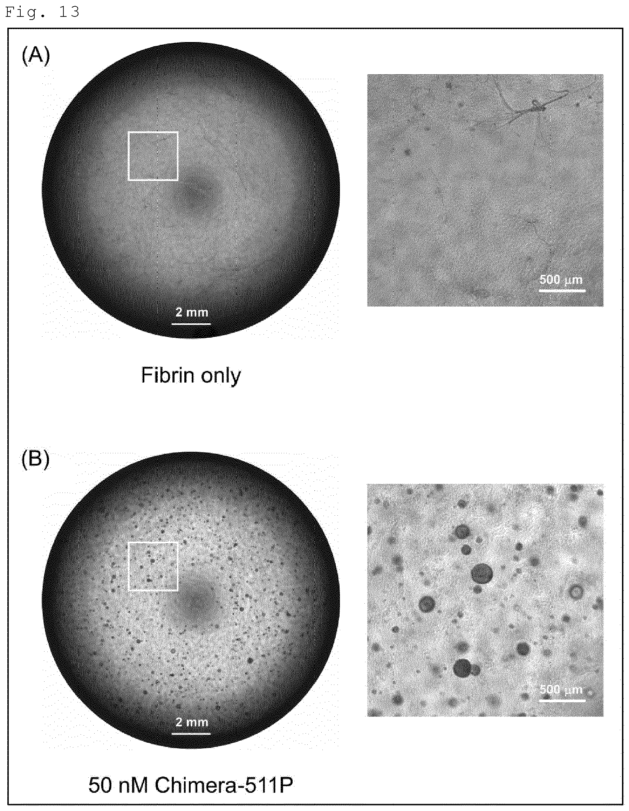

The images of the hiPS cells on day 7 of culture observed with an all-in-one fluorescence microscope are shown in Fig. 13. Fig. 13A shows images of the negative control (Fibrin only). Fig. 13B shows images of the hiPS cells cultured in the fibrin gel incorporating 50 nM Chimera-5 11P. On the left are images of the entire wells, and on the right are enlarged images of the boxed regions in the images on the left. As shown in Fig. 13A, no cell clusters were observed on day 7 of culture in the negative control. In contrast, a large number of cell clusters of grown hiPS cells were observed in the culture in the fibrin gel containing 50 nM Chimera-5 11P as shown in Fig. 13B.

### Example 9: Production, purification, and activity evaluation of chimeric molecules composed of fibrinogen and a laminin isoform other than laminin 511

### (1) Expression vectors for chimeric proteins having human fibrinogen fused to an integrin-binding domain of laminin 111, laminin 121, laminin 211, laminin 221, laminin 311, laminin 321, laminin 332, laminin 411, laminin 421, or laminin 521

An expression vector for a chimeric protein of a human laminin α1 chain with a C-terminal 10×His tag, designated herein as Chimera-α1 (mouse Ig-κ chain V-J2-C signal peptide/FBGβ (Gln³¹-Asn¹⁹⁴)/LMα1 (Leu²⁰⁹⁹-Pro²⁶⁸³)/10×His), was prepared using the previously described expression vector for a human laminin α1E8 fragment (Hiroyuki Ido et al. J Biol Chem 283:28149-28157, 2008) as a template, according to the method described in Example 1 (2). Similarly, expression vectors for a chimeric protein of a human laminin α2 chain, designated herein as Chimera-α2 (mouse Ig-κ chain V-J2-C signal peptide/FBGβ (Gln³¹-Asn¹⁹⁴)/LMα2 (Ile²¹²⁷-Asp²⁷²⁰)/10×His), a chimeric protein of a human laminin α3 chain, designated herein as Chimera-α3 (mouse Ig-κ chain V-J2-C signal peptide/FBGβ (Gln³¹-Asn¹⁹⁴)/LMα3 (Met²³⁷⁰-Leu²⁹³⁷)/10×His), and a chimeric protein of a human laminin α4 chain, designated herein as Chimera-α4 (mouse Ig-κ chain V-J2-C signal peptide/FBGβ (Gln³¹-Asn¹⁹⁴)/LMα4 (Ile⁸¹⁵-Leu¹⁴¹²)/10×His), were prepared using the previously described expression vectors for a human laminin α2E8 fragment (Hiroyuki Ido et al. J Biol Chem 283:28149-28157, 2008), a human laminin α3E8 fragment (Takamichi Miyamzaki et al. Nature Commun. 3:1236, 2012), and a human laminin α4E8 fragment (Ryo Ohta et al. Sci Rep 6:35680, 2016) as templates, respectively. For Chimera-α1 and Chimera-α2, expression vectors for chimeric proteins in which Asn194 of the human fibrinogen β chain was replaced with glutamine (Gln) were prepared in order to avoid the generation of an N-glycosylation consensus sequence (Asn-Xaa-Ser) at the junction between the human fibrinogen β chain ("FBGβ (Met¹-Gln⁴⁹¹)") and the human laminin α1 or α2 chain.

An expression vector for a chimeric protein of a human laminin β2 chain, designated herein as Chimera-β2 (mouse Ig-κ chain V-J2-C signal peptide/FBGα (Ala²⁰-His¹⁵¹)/LMβ2 (Leu¹⁷⁷³-Gln¹⁷⁹⁸)), was prepared using the previously described expression vector for a human laminin β2E8 fragment (Yukimasa Taniguchi et al. J Biol Chem 284:7820-7831, 2009) as a template, according to the method described in Example 1. Similarly, expression vectors for a chimeric protein of a human β3 chain, designated herein as Chimera-β3 (mouse Ig-κ chain V-J2-C signal peptide/FBGα (Ala²⁰-His¹⁵¹)/LMβ3 (Leu¹¹⁴⁷-Lys¹¹⁷²)), and a chimeric protein of a human γ2 chain, designated herein as Chimera-y2 (mouse Ig-κ chain V-J2-C signal peptide/FBGα (Ala²⁰-His¹⁵¹)/LMγ2 (Ile¹¹⁶³-Gln¹¹⁹³)), were prepared using the previously described expression vectors for a human laminin β3E8 fragment (Takamichi Miyamzaki et al. Nature Commun 3:1236, 2012) and a human laminin γ2E8 fragment (Takamichi Miyamzaki et al. Nature Commun 3:1236, 2012) as templates, respectively.

### (2) Expression and purification of chimeric proteins having human fibrinogen fused to an integrin-binding domain of laminin 111, laminin 121, laminin 211, laminin 221, laminin 311, laminin 321, laminin 332, laminin 411, laminin 421, or laminin 521 (Chimera-111, Chimera-121, Chimera-211, Chimera-221, Chimera-311, Chimera-321, Chimera-332, Chimera-411, Chimera-421, and Chimera-521)

Chimera-111 was expressed in 293-F cells transfected with the expression vectors for Chimera-α1, Chimera-β1, and Chimera-γ1 according to the method described in Example 1 (3). Similarly, Chimera-121, Chimera-211, Chimera-221, Chimera-311, Chimera-321, Chimera-332, Chimera-411, Chimera-421, Chimera-511, and Chimera-521 were expressed in 293-F cells transfected with appropriate combinations of the expression vectors for Chimera-α1, Chimera-α2, Chimera-α3, Chimera-α4, Chimera-α5, Chimera-β1, Chimera-β2, Chimera-β3, Chimera-γ1, and Chimera-γ2 in accordance with their laminin subunit composition.

Seventy-two hours after the start of culture, the culture media were collected and centrifuged to harvest the culture supernatants. Chimeric proteins were purified from the culture supernatants by a combination of affinity chromatography based on binding to the 10×His tag fused to the C-terminus of the Chimera-α chain and gel filtration chromatography on Superose 6 Increase 10/300GL (GE Healthcare) according to the method described in Example 1 (3). The purified chimeric proteins were sterilized with a 0.22-µm disk syringe filter (Merck Millipore, SLGV033RS) and stored at -80°C.

### (3) SDS-PAGE analysis of Chimera-111, Chimera-121, Chimera-211, Chimera-221, Chimera-311, Chimera-321, Chimera-332, Chimera-411, Chimera-421, and Chimera-521

### (3-1) Confirmation of chimeric protein expression

The culture medium of the 293-F cells transfected with the expression vectors for each chimeric protein was collected 72 hours after the start of culture. The culture supernatant was analyzed for the expression of the chimeric protein of interest by SDS-PAGE. A mixture of 5 µL of the culture supernatant and 1.3 µL of 5× SDS sample treatment buffer was treated at 95°C for 5 minutes. This mixture was applied on a 5-20% gradient polyacrylamide gel (ATTO, #2331830) and electrophoresed at 15 mA for 115 minutes. Electrophoresis was performed under non-reducing conditions according to the Laemmli method, using a buffer containing 25 mM Tris, 192 mM glycine, and 0.1% sodium dodecyl sulfate. After electrophoresis, the separated proteins were transferred to a polyvinylidene difluoride membrane. The chimeric proteins transferred on the membrane were detected using an antibody (Penta-His HRP conjugate; QIAGEN, #34460) against the 10×His tag fused to the C-terminus of Chimera-α1, Chimera-α2, Chimera-α3, Chimera-α4, and Chimera-aS.

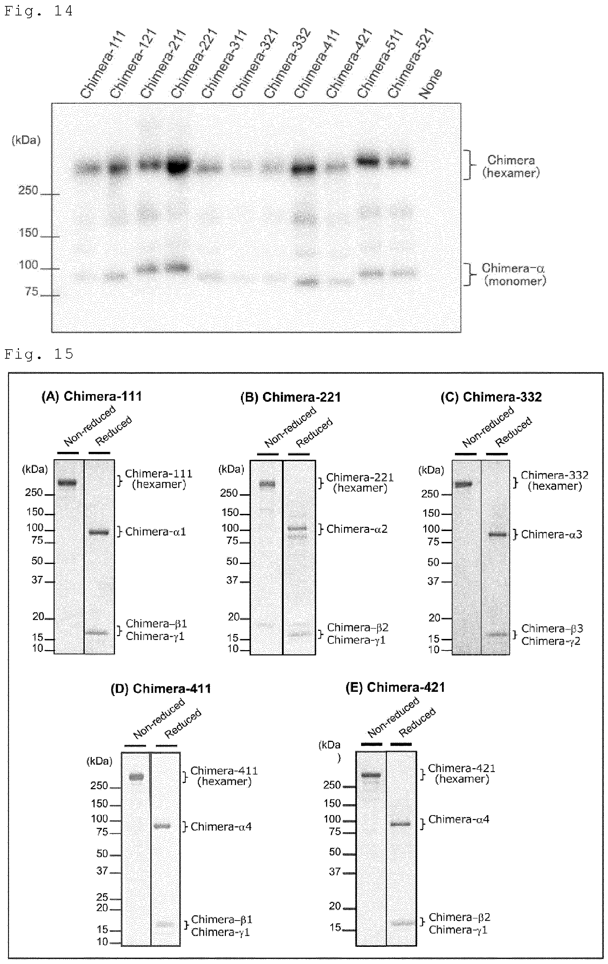

The results are shown in Fig. 14. The SDS-PAGE analysis of each culture supernatant showed a band at the position higher than that of the 250-kDa molecular weight marker, corresponding to a hexameric form of the chimeric protein of interest. In addition, another band was detected at the position of the 100-kDa molecular weight marker, corresponding to a small amount of Chimera-α1, Chimera-α2, Chimera-α3, Chimera-α4, or Chimera-α5. These results confirmed that each chimeric protein was expressed as a hexamer as with Chimera-511.

### (3-2) Purity determination of purified chimeric proteins

Purified Chimera-111, Chimera-221, Chimera-332, Chimera-411, and Chimera-421 were each applied at 1.2 µg/well on a 5-20% gradient polyacrylamide gel (ATTO, #2331830) and electrophoresed at 20 mA for 75 minutes. After electrophoresis, the gel was stained with Quick-CBB (FUJIFILM Wako Pure Chemical Corporation, #299-50101) to visualize the separated proteins.

The results are shown in Fig. 15. The non-reducing SDS-PAGE analysis for each chimeric protein showed a single band at the position higher than that of the 250-kDa molecular weight marker, corresponding to a hexameric form of the chimeric protein of interest. On the other hand, the reducing SDS-PAGE analysis showed a band at the position of the 100-kDa molecular weight marker, corresponding to Chimera-α 1, Chimera-α2, Chimera-α3, or Chimera-α4, and another band at the position slightly higher than that of the 15-kDa molecular weight marker, corresponding to Chimera-β1, Chimera-β2, Chimera-β3, Chimera-γ1, or Chimera-γ2. These results confirmed that each chimeric protein was purified in high purity.

### (4) Measurement of fibrinogen-binding activity and integrin-binding activity of Chimera-111, Chimera-221, Chimera-332, and Chimera-421

### (4-1) Fibrinogen-binding activity

The fibrinogen-binding activity of purified Chimera-111, Chimera-221, Chimera-332, and Chimera-421 was measured according to the method described in Example 1 (6). Human fibrinogen was diluted to a final concentration of 100 nM in HBS (pH 7.4) and added to 96-well plates at 50 µL/well. The plates were incubated with gentle agitation at room temperature overnight for coating. The human fibrinogen-coated 96-well plates were washed with 200 µL/well of 1% Skim/TBST. Next, 1% Skim/TBST was added at 200 µL/well, and the plates were incubated with agitation on a shaker at room temperature for 1 hour for blocking. The plates were washed once with TBST, and a 0.5 NIH unit/mL thrombin solution was added to the plates at 25 µL/well. Each chimeric protein was diluted to final concentrations of 0.3125 nM, 0.625 nM, 1.25 nM, 2.5 nM, 5 nM, and 10 nM in HBS (pH 7.4), added at 25 µL/well, and allowed to react with agitation using the shaker at room temperature for 1 hour. For controls, HBS (pH 7.4) was added at 25 µL/well instead of the thrombin solution.

The amount of each chimeric protein bound to fibrinogen was quantified using an antibody against the 10×His tag fused to the C-terminus of the Chimera-α chain (Penta-His HRP conjugate; QIAGEN, #34460; hereinafter referred to as "anti-His-tag antibody"). After washing three times with 200 µL/well of TBST, the anti-His tag antibody diluted at 1:3000 with 1% Skim/TBST was added to the plates at 50 µL/well and allowed to react with agitation using the shaker at room temperature for 1 hour. Then, the plates were washed three times with 200 µL/well of TBST. o-phenylenediamine was dissolved at a final concentration of 0.4 mg/mL in 25 mM citric acid/50 mM Na₂HPO₄ buffer containing 0.04% H₂O₂, added at 50 µL/well, and allowed to react for 2 minutes. The reaction was stopped by addition of 2.5 M H₂SO₄, and the absorbance at 490 nm of the chromogenic substrate was measured with a microplate reader.

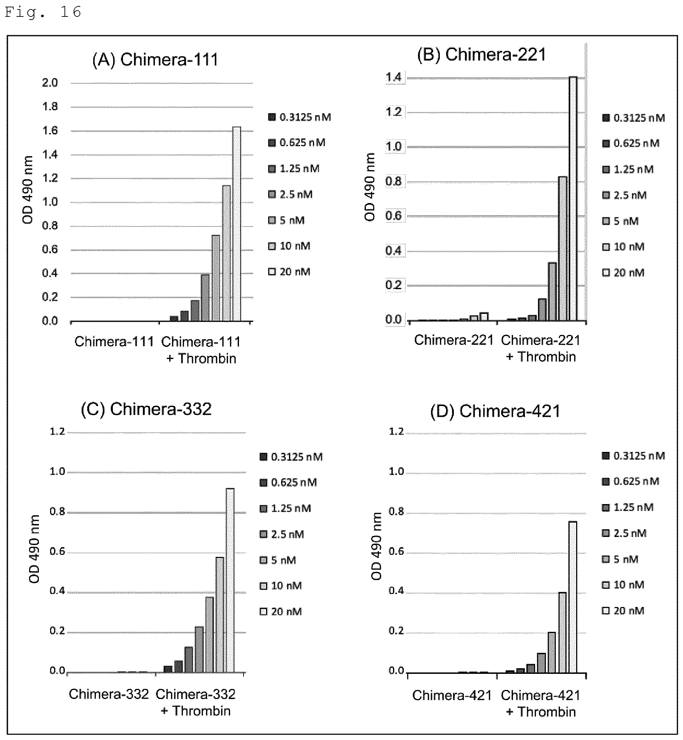

The results are shown in Fig. 16. The amount of each chimeric protein bound to fibrinogen increased in a concentration-dependent manner in the presence of thrombin, but none of the chimeric proteins bound to fibrinogen in the absence of thrombin. The results indicate that, as with Chimera-511, each chimeric protein retains thrombin-dependent fibrinogen-binding activity.

### (4-2) Integrin-binding activity

Since each chimeric protein contains an integrin-binding domain of laminin, its integrin-binding activity can be used to quantify the amount of the chimeric protein bound to fibrinogen on 96-well plates in a thrombin-dependent manner. That is, laminin-binding integrins can be used instead of an anti-His-tag antibody to quantify the amount of the chimeric protein bound to fibrinogen. Among laminin-binding integrins, laminin-111 and laminin-221 bind strongly to α7X2β1 integrin, whereas laminin-332 and laminin-421 bind strongly to α6β1 integrin (Yukimasa Taniguchi et al. J Biol Chem 284:7820-7831, 2009; Ryoko Nishiuchi et al. Matrix Biol 25:189-197, 2006; Taichi Ishikawa et al. Matrix Biol 38:69-83, 2014). Based on these findings, the amount of Chimera-111 or Chimera-221 bound to fibrinogen was quantified using α7X2β1 integrin, and the amount of Chimera-332 or Chimera-421 bound to fibrinogen was quantified using α6β1 integrin.

The α7X2β1 integrin and α6β1 integrin were produced by the method of Ido et al. (Hiroyuki Ido et al. J Biol Chem 282(15):11144-11154, 2007). Purified Chimera-111, Chimera-221, Chimera-332, and Chimera-421 were added to fibrinogen-coated 96-well plates and allowed to bind to the fibrinogen on the plates according to the method described in the above (4-1). The amount of the integrin bound to each chimeric protein was quantified according to the method described in Example 1 (5-2). More specifically, the two different integrins were separately diluted to a final concentration of 30 nM in 0.1% BSA/TBST containing 1 mM MnCl₂ or 10 mM EDTA, added to the plates at 50 µL/well, and allowed to react with agitation using the shaker at room temperature for 1 hour. Then, the plates were washed three times with 200 µL/well of 1 mM MnCl₂/0.1% BSA/TBST or 10 mM EDTA/0.1% BSA/TBST. A 1.5 µg/mL solution of a biotin-labeled Velcro antibody (prepared as described in Takagi, J., Erickson, H. P. and Springer, T. A. (2001) Nat. Struct. Biol. 8, 412-416) in 1 mM MnCl₂/0.1% BSA/TBST was added at 50 µL/well and allowed to react with agitation using the shaker at room temperature for 30 minutes. Then, the plates were washed three times with 200 µL/well of 1 mM MnCl₂/0.1% BSA/TBST. A 0.53 µg/mL solution of streptavidin-horseradish peroxidase (Thermo Fisher Scientific, #21126) in 1 mM MnCl₂/0.1% BSA/TBST was added at 50 µL/well and allowed to react with agitation using the shaker at room temperature for 15 minutes. Then, the plates were washed three times with 200 µL/well of 1 mM MnCl₂/0.1% BSA/TBST. o-phenylenediamine (OPD; FUJIFII,M Wako Pure Chemical Corporation, #615-28-1) was dissolved at a final concentration of 0.4 mg/mL in 25 mM citric acid/50 mM Na₂HPO₄ buffer containing 0.04% H₂O₂, added at 50 µL/well, and allowed to react for 2 to 5 minutes. The reaction was stopped by addition of 2.5 M H₂SO₄, and the absorbance at 490 nm of the chromogenic substrate was measured with a microplate reader (Molecular Devices EMax).

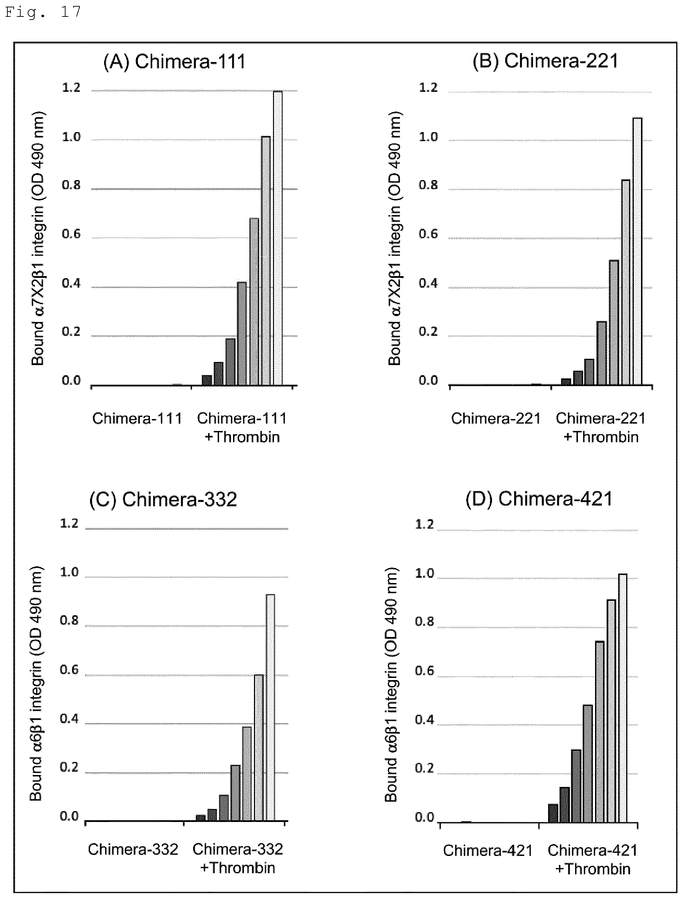

The results are shown in Fig. 17. All the tested chimeric proteins bound strongly to integrin in the presence of 1 mM MnCl₂, but none of them bound to integrin in the presence of 10 mM EDTA. On the other hand, when the chimeric proteins were added to fibrinogen-coated 96-well plates in the absence of thrombin, no integrin binding was detected, which was consistent with the case using the anti-His-tag antibody for detection. These results show that these chimeric proteins bind to fibrinogen in a thrombin-dependent manner regardless of the isoform of laminin chimerized with fibrinogen, and retain integrin-binding activity even in a state of being bound to fibrinogen.

### Example 10: Production, purification, and activity evaluation of perlecan-fused chimeric molecules composed of fibrinogen and a laminin isoform other than laminin 511

### (1) Construction of expression vectors for Chimera-α1, Chimera-α2, Chimera-α3, and Chimera-α4 each fused to human perlecan domain 1 (D1)

An expression vector for a chimeric protein having human perlecan D1 fused to the C-terminus of Chimera-α1 (hereinafter referred to as "Chimera-α1(+P)") was constructed in the same manner as described in Example 7 (1), except for using, as a template, the previously described expression vector for a chimeric protein having human perlecan D1 fused to the C-terminus of a laminin α1E8 fragment (WO2018/088501A1), which was constructed according to the procedure described in WO2014/199754A1. Similarly, expression vectors for chimeric proteins having human perlecan D1 fused to the C-terminus of Chimera-α2, Chimera-α3, or Chimera-α4 (hereinafter referred to as "Chimera-α2(+P)", "Chimera-α3(+P)", and "Chimera-α4(+P)") were constructed using the previously described expression vectors for a laminin α2E8 fragment, a laminin α3E8 fragment, and a laminin α4E8 fragment each C-terminally fused to human perlecan D1 (WO2018/088501A1).

### (2) Expression and purification of perlecan-fused Chimera-111, Chimera-121, Chimera-211, Chimera-221, Chimera-311, Chimera-321, Chimera-332, Chimera-411, Chimera 421, and Chimera-521

Perlecan-fused Chimera-111 (hereinafter referred to as "Chimera-111P") was expressed in 293-F cells transfected with the expression vectors for Chimera-α1(+P), Chimera-β1, and Chimera-γ1 according to the method described in Example 7 (2). Similarly, perlecan-fused Chimera-121, Chimera-211, Chimera-221, Chimera-311, Chimera-321, Chimera-332, Chimera-411, Chimera-421, and Chimera-521 (hereinafter referred to as "Chimera-121P", "Chimera-211P", "Chimera-221P", "Chimera-311P", "Chimera-321P", "Chimera-332P", "Chimera-411P", "Chimera-421P", and "Chimera-521P", respectively) were expressed in 293-F cells transfected with appropriate combinations of the expression vectors for Chimera-α1(+P), Chimera-α2(+P), Chimera-α3(+P), Chimera-α4(+P), Chimera-α5(+P), Chimera-β1, Chimera-β2, Chimera-β3, Chimera-γ1, and Chimera-y2 in accordance with their laminin subunit composition according to the method described in Example 7 (2). Seventy-two hours after the start of culture, the culture media were collected and centrifuged to harvest the culture supernatants.

Heparan sulfate chains attached to the perlecan D1 bind strongly to an anion exchanger having a quaternary ammonium group. This property can be used for purification of perlecan D1-containing chimeric proteins simply by anion exchange chromatography, instead of a combination of affinity chromatography based on binding to the 10×His tag and gel filtration chromatography. The specific procedure was as follows. The collected culture medium (300 mL) was centrifuged at 500 × g for 10 minutes, and the supernatant was further centrifuged at 10,000 × g for 30 minutes to remove remaining cells and insoluble matter. Two HiTrap (registered trademark) Q Fast Flow columns (5 mL) (Cytiva, 17515601) were connected to an AKTA avant 25 system, and the supernatant was passed through the columns at a flow rate of 2.5 mL/min. The columns were washed with 100 mL of 20 mM Tris-HCl (pH 8.0) containing 2 mM NaCl at a flow rate of 5 mL/min, followed by elution of the adsorbed protein with 20 mM Tris-HCl (pH 8.0) and 20 mM Tris-HCl (pH 8.0) containing 1 M NaCl. The eluted fractions containing the protein of interest were identified by SDS-PAGE analysis and then pooled. The NaCl concentration in the fraction pool was adjusted to about 500 mM with 20 mM Tris-HCl (pH 8.0). Next, one HiTrap Q HP column (5 mL) (Cytiva, 17115401) was connected to an AKTA avant 25 system, and the fraction pool was passed through the column at a flow rate of 2.5 mL/min. The column was washed with 50 mL of 20 mM Tris-HCl (pH 8.0) containing 450 to 500 mM NaCl at a flow rate of 2.5 mL/min, and the adsorbed protein was eluted with 20 mM Tris-HCl (pH 8.0) containing 2 M NaCl. The eluted fractions containing the protein of interest were identified by SDS-PAGE analysis and then pooled. The fraction pool was dialyzed against HBS(-) (pH 7.4). The purified product obtained through dialysis was sterilized with a 0.22-µm disk syringe filter (Merck Millipore, SLGVJ13SL). The protein concentration was determined with the Pierce BCA Protein Assay Kit (Thermo Fisher Scientific, 23227), and the purified product was stored at -80°C.

### (3) SDS-PAGE analysis of Chimera-111P, Chimera-121P, Chimera-211P, Chimera-221P, Chimera-311P, Chimera-321P, Chimera-332P, Chimera-411P, Chimera-421P, and Chimera-521P

### (3-1) Confirmation of perlecan-fused chimeric protein expression

The culture medium of the 293-F cells transfected with the expression vectors for each chimeric protein was collected 72 hours after the start of culture. The culture supernatant was analyzed for the expression of the chimeric protein of interest by SDS-PAGE under non-reducing conditions. A mixture of 16 µL of the culture supernatant and 4 µL of 5× SDS sample treatment buffer was treated at 95°C for 5 minutes. This mixture was applied on a 5-20% gradient polyacrylamide gel (ATTO, #2331830) and electrophoresed at 20 mA for 75 minutes. After electrophoresis, the separated proteins were transferred to a polyvinylidene difluoride membrane. The chimeric proteins transferred on the membrane were detected using an antibody (Penta-His HRP conjugate; QIAGEN, #34460) against the 10×His tag fused to the C-terminus of Chimera-α1P, Chimera-α2P, Chimera-α3P, Chimera-α4P, and Chimera-α5P.

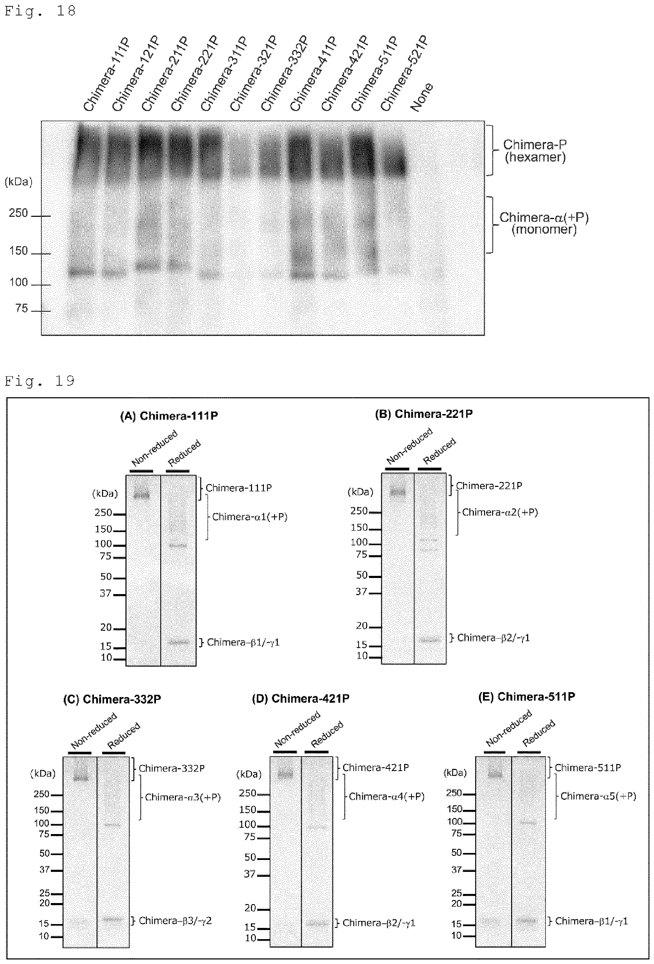

The results are shown in Fig. 18. The non-reducing SDS-PAGE analysis of each culture supernatant showed a slightly broad, single band at the position higher than that of the 250-kDa molecular weight marker. These results confirmed that each perlecan-fused chimeric protein was expressed as a hexamer as designed. The expression levels of the perlecan-fused chimeric proteins were different with the isoform of laminin. The chimeric proteins containing a laminin α1, α2, or α4 chain (i.e., Chimera-111P, Chimera-121P, Chimera-211P, Chimera-221P, Chimera-411P, and Chimera-421P) seemed to be more highly expressed than the chimeric proteins containing a laminin α3 or α5 chain (i.e., Chimera-311P, Chimera-321P, Chimera-332P, Chimera-511P, and Chimera-521P).

### (3-2) Purity determination of purified chimeric proteins

Purified Chimera-111P, Chimera-221P, Chimera-332P, Chimera-421P, and Chimera-5 11P were each applied at 1.2 µg/well on a 5-20% gradient polyacrylamide gel (ATTO, #2331830) and electrophoresed at 20 mA for 75 minutes. After electrophoresis, the gel was stained with Quick-CBB (FUJIFILM Wako Pure Chemical Corporation, #299-50101) to visualize the separated proteins.

The results are shown in Fig. 19. The non-reducing SDS-PAGE analysis for each chimeric protein showed a band at the position higher than that of the 250-kDa molecular weight marker, corresponding to a hexameric form of the chimeric protein of interest. On the other hand, the reducing SDS-PAGE analysis showed a diffuse band covering a range of molecular weight of 150 kDa to 250 kDa or more, corresponding to Chimera-α1(P+), Chimera-α2(P+), Chimera-α3(P+), Chimera-α4(P+), or Chimera-α5(P+). Proteins containing heparan sulfate chains (heparan sulfate proteoglycans) are characterized in that their heparan sulfate chains have a non-uniform length and degree of sulfation, which result in the appearance of a diffuse broad band in SDS-PAGE analysis as previously reported (Shaoliang Li et al. J Biol Chem 285:36645-36655, 2010). The appearance of a diffuse band covering a range of molecular weight of 150 kDa to 250 kDa or more in the reducing SDS-PAGE analysis indicates that a sufficient amount of heparan sulfate chains were attached to the perlecan D1 of Chimera-α1(+P), Chimera-α2(+P), Chimera-α3(+P), Chimera-α4(+P), or Chimera-α5(+P). The reducing SDS-PAGE analysis showed another band at the position slightly higher than that of the 15-kDa molecular weight marker, corresponding to Chimera-β1, Chimera-β2, Chimera-β3, Chimera-γ1, or Chimera-γ2. It is noted that the sharp band detected at the position of the 100-kDa molecular weight marker in the reducing SDS-PAGE analysis corresponds to a Chimera-α(+P) chain in which the perlecan D1 was cut off, which was confirmed by western blotting using an antibody that specifically recognizes the perlecan D1.

### (4) Measurement of fibrinogen-binding activity and integrin-binding activity of Chimera-111P, Chimera-221P, Chimera-332P, and Chimera-421P

The fibrinogen-binding activity and integrin-binding activity of purified Chimera-111P, Chimera-221P, Chimera-332P, and Chimera-421P were measured according to the method described in Example 1 (6). Human fibrinogen was diluted to a final concentration of 100 nM in HBS (pH 7.4) and added to 96-well plates at 50 µL/well. The plates were incubated with gentle agitation at room temperature overnight for coating. The fibrinogen-coated 96-well plates were washed with 200 µL/well of 1% Skim/TBST. Next, 1% Skim/TBST was added at 200 µL/well, and the plates were incubated with agitation on a shaker at room temperature for 1 hour for blocking. The plates were washed once with TBST, and a 0.5 NIH unit/mL thrombin solution was added to the plates at 25 µL/well. Each chimeric protein was diluted to final concentrations of 0.3125 nM, 0.625 nM, 1.25 nM, 2.5 nM, 5 nM, and 10 nM in HBS (pH 7.4), added at 25 µL/well, and allowed to react with agitation using the shaker at room temperature for 1 hour. For controls, HBS was added at 25 µL/well instead of the thrombin solution.

Since the perlecan-fused chimeric proteins contain an integrin-binding domain of laminin, the amount of each chimeric protein bound to fibrinogen was measured using its integrin-binding activity as an indicator. More specifically, the amount of Chimera-111P or Chimera-221P bound to fibrinogen was quantified using α7X2β1 integrin, and the amount of Chimera-332P or Chimera-421P bound to fibrinogen was quantified using α6β1 integrin according to the method described in Example 9 (4-2).

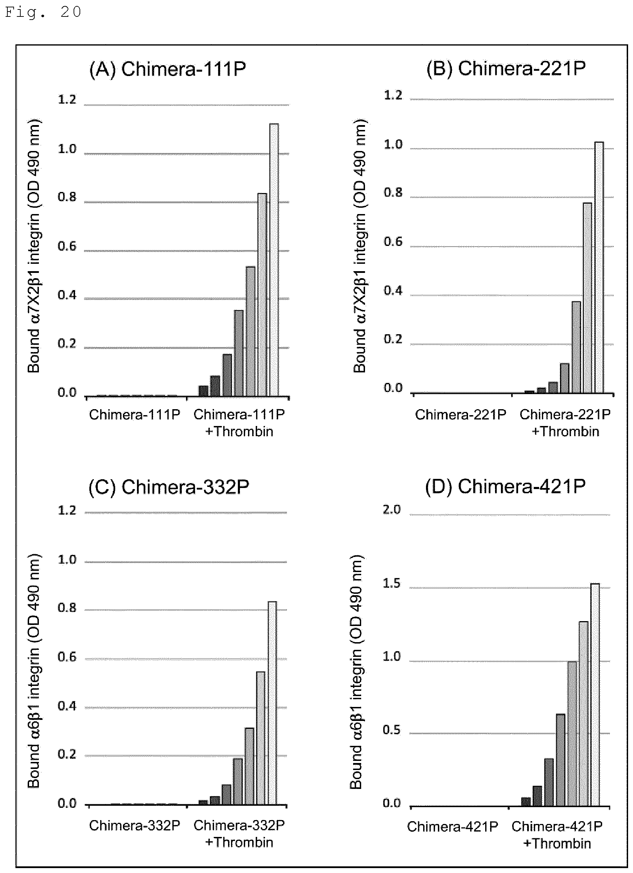

The results are shown in Fig. 20. All the tested perlecan-fused chimeric proteins bound strongly to integrin in the presence of 1 mM MnCl₂, but none of them bound to integrin in the presence of 10 mM EDTA. On the other hand, when the perlecan-fused chimeric proteins were added to fibrinogen-coated 96-well plates in the absence of thrombin, no integrin binding was detected. These results show that these perlecan-fused chimeric proteins bind to fibrinogen in a thrombin-dependent manner regardless of the isoform of laminin chimerized with fibrinogen, and retain integrin-binding activity even in a state of being bound to fibrinogen.

### (5) Measurement of growth factor-binding activity of Chimera-111P, Chimera-221P, Chimera-332P, and Chimera-421

Perlecan is known to trap various growth factors via its heparan sulfate chains (Shaoliang Li et al. J Biol Chem 285:36645-36655, 2010). In order to confirm that the perlecan-fused chimeric proteins retain growth factor-binding activity, the binding activity of purified perlecan-fused chimeric proteins to proactivin A, which has been reported to bind to heparan sulfate chains, was measured.

### (5-1) Expression and purification of proactivin A

Proactivin A was expressed in 293-F cells transfected with an expression vector for a full-length human proactivin with an N-terminal 6×His tag. Specifically, 360 µg of this expression vector was transfected into 3 × 10⁸ 293-F cells (1.0 × 10⁶ cells/mL) using the transfection reagent 293fectin and Opti-MEM, and the culture medium was collected after 72 hours of culture.

The collected culture medium was centrifuged at 500 × g for 10 minutes, and the supernatant was further centrifuged at 12,000 × g for 30 minutes to remove remaining cells and insoluble matter. To the culture supernatant, imidazole, Pefabloc SC PLUS (Roche, #11873628001), and sodium azide were added at final concentrations of 5 mM, 1 mM, and 0.02% (w/v), respectively, and the mixture was stirred. One cOmplete His-Tag Purification Column in a prepacked format (1 mL) (Roche, #6781535001) was connected to an AKTA avant 25 system, and the supernatant was passed through the column at a flow rate of 1 mL/min. The column was washed with 10 mL of 50 mM Tris-HCl (pH 8.0) containing 200 mM NaCl at a flow rate of 1 mL/min, and the adsorbed protein was eluted with 50 mM Tris-HCl (pH 8.0) containing 250 mM imidazole and 200 mM NaCl. The eluted fractions containing the protein of interest were identified by SDS-PAGE analysis and then pooled. The fraction pool was diluted 10-fold with 25 mM Tris-HCl (pH 8.0). Next, one RESOURCE Q (1 mL) (Cytiva, 17117701) was connected to an AKTA avant 25 system, and the fraction pool was passed through the column at a flow rate of 2 mL/min. The column was washed with 10 mL of 25 mM Tris-HCl (pH 8.0) at a flow rate of 4 mL/min, and the adsorbed protein was eluted with 25 mM Tris-HCl (pH 8.0) containing 1 M NaCl. The eluted fractions containing the protein of interest were identified by SDS-PAGE analysis.

The relevant fractions were concentrated using Amicon Ultra-4 Centrifugal Filter Units - 10,000 NMWL (Millipore, UFC801008). Next, the concentrate was subjected to gel filtration chromatography on a Superose 6 Increase 10/300GL (GE Healthcare), followed by elution with D-PBS(-) (Nacalai Tesque, 14249-95) at a flow rate of 0.5 mL/min. The eluted fractions containing the protein of interest were identified by SDS-PAGE analysis. The purified product was sterilized with a 0.22-µm disk syringe filter (Merck Millipore, SLGVJ13SL). The protein concentration was determined with the Pierce BCA Protein Assay Kit (Thermo Fisher Scientific, 23227), and the purified product was stored at -80°C.

### (5-2) Proactivin A binding assay

Chimera-111P, Chimera-221P, Chimera-332P, Chimera-421P, and Chimera-5 11P were separately diluted to final concentrations of 0.31 nM, 0.63 nM, 1.25 nM, 2.5 nM, 5 nM, 10 nM, and 20 nM in HBS (pH 7.4). The diluted solutions were added to 96-well plates (Thermo Fisher Scientific, #442404) at 50 µL/well. The plates were incubated with gentle agitation at 4°C overnight for coating.

The perlecan-fused chimeric protein-coated 96-well plates were washed with 200 µL/well of 1% BSA/TBST. Next, 1% BSA/TBST was added at 200 µL/well, and the plates were incubated with agitation on a shaker at room temperature for 1 hour for blocking. The plates were washed once with 1% BSA/TBST. 1% BSA/TBST containing 30 nM proactivin A was added to the plates at 50 µL/well and allowed to react with agitation using the shaker at room temperature for 1 hour. Then, the plates were washed three times with 200 µL/well of TBST. A 0.5 µg/mL solution of mouse anti-Activin A βA Subunit (R&D SYSTEMS, MAB3381) in 1% BSA/TBST was added at 50 µL/well and allowed to react with agitation using the shaker at room temperature for 1 hour. Then, the plates were washed three times with 200 µL/well of TBST. A 0.4 µg/mL solution of donkey anti-mouse IgG pAb/HRP (Jackson ImmunoResearch Laboratories, 715-035-150) in 1% BSA/TBST was added at 50 µL/well and allowed to react with agitation using the shaker at room temperature for 1 hour. Then, the plates were washed three times with 200 µL/well of TBST. o-phenylenediamine was dissolved at a final concentration of 0.4 mg/mL in 25 mM citric acid/50 mM Na₂HPO₄ buffer containing 0.04% H₂O₂, added at 50 µL/well, and allowed to react for 10 minutes. The reaction was stopped by addition of 2.5 M H₂SO₄, and the absorbance at 490 nm of the chromogenic substrate was measured with a microplate reader.

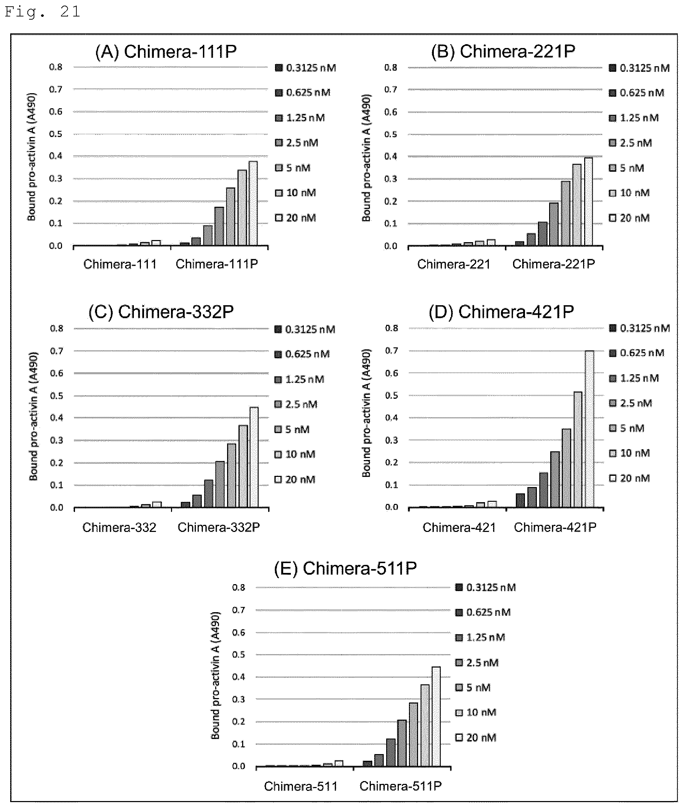

The results are shown in Fig. 21. The perlecan-fused chimeric proteins on the plates bound to proactivin A in a concentration dependent manner, while the chimeric proteins without a perlecan domain showed no significant binding to proactivin A. These results indicate that proactivin A was bound to the perlecan-fused chimeric proteins via the heparan sulfate chains attached to the perlecan domain.

### Example 11: Production of chimeric molecules having different combinations of human fibrinogen Aα, Bβ, and y chains and human laminin α5, β1, and γ1 chains (1) Construction of expression vectors for chimeric proteins having different combinations of human fibrinogen Aα, Bβ, and y chains and human laminin α5, β1, and γ1 chains

Chimeric proteins composed of laminin (a heterotrimer of α, β, and y chains) and fibrinogen (a heterotrimer of Aα, Bβ, and γ chains) can be produced in the following three different combinations of laminin and fibrinogen subunit chains.
Combination-1 (PBGα-LMα/PBGγ-LMβ/FBGβ-LMγ)
Combination-2 (FBGγ-LMα/FBGβ-LMβ/FBGα-LMγ)
Combination-3 (PBOPβ-LMα/FBGα-LMβ/FBGγ-LMγ)

In order to confirm that these three different chimeric proteins could be expressed, expression vectors for each chimeric protein were constructed. It is noted that the expression vectors for Combination-3 were the same as those described in Example 9.

### Construction of expression vectors for Combination-1

Expression vectors for Chimera-α5 consisting of mouse Ig-κ chain V-J2-C signal peptide/FBGα (Ala²⁰-His¹⁵¹)/LMα5 (Ile²⁷¹⁶-Ala³³¹⁰)/10×His, Chimera-β1 consisting of mouse Ig-κ chain V-J2-C signal peptide/FBGγ (Tyr²⁷-Ser¹³²)/LMβ1 (Leu¹⁷⁶¹-Leu¹⁷⁸⁶), and Chimera-γ1 consisting of mouse Ig-κ chain V-J2-C signal peptide/FBGβ (Gln³¹-Asn¹⁹⁴)/LMγ1 (Ile¹⁵⁷⁹-Pro¹⁶⁰⁹) were constructed as follows. First, the expression vectors for mouse Ig-κ chain V-J2-C signal peptide/LMα5 (Ile²⁷¹⁶-Ala³³¹⁰)/10×His, mouse Ig-κ chain V-J2-C signal peptide/LMβ1 (Leu¹⁷⁶¹-Leu¹⁷⁸⁶), mouse Ig-κ chain V-J2-C signal peptide/LMγ1 (Ile¹⁵⁷⁹-Pro¹⁶⁰⁹), mouse Ig-κ chain V-J2-C signal peptide/FBGβ (Gln³¹-Asn¹⁹⁴)/LMα5 (Ile²⁷¹⁶-Ala³³¹⁰)/10×His, mouse Ig-κ chain V-J2-C signal peptide/FBGα (Ala²⁰-His¹⁵¹)/LMβ1 (Leu¹⁷⁶¹-Leu¹⁷⁸⁶), and mouse Ig-κ chain V-J2-C signal peptide/FBGγ (Tyr²⁷-Ser¹³²)/LMγ1 (Ile¹⁵⁷⁹-Pro¹⁶⁰⁹) described in Example 1 were used as templates to amplify DNA fragments encoding mouse Ig-κ chain V-J2-C signal peptide/FBGα (Ala²⁰-His¹⁵¹), LMα5 (Ile²⁷¹⁶-Ala³³¹⁰)/10×His, mouse Ig-κ chain V-J2-C signal peptide/FBGγ (Tyr⁷-Ser¹³²), LMβ1 (Leu¹⁷⁶¹-Leu¹⁷⁸⁶), mouse Ig-κ chain V-J2-C signal peptide/FBGβ (Gln³¹-Asn¹⁹⁴), and LMγ1 (Ile¹⁵⁷⁹-Pro¹⁶⁰⁹) by PCR using their respective primer sets shown below. The reverse primer of each primer set has a sequence used for extension PCR at the 5' end.
(xxi) Primer set for amplification of mouse Ig-κ chain V-J2-C signal peptide/FBGα (Ala²⁰-His¹⁵¹)
   5'-CGGGACTTTCCAAAATGTCGTAACAACTCCGCCCCATTGAC-3' (forward, SEQ ID NO: 27) 
5’-CAATGAGCTCTCGCACGCGGCCAATATGCTGTACTTTTTCTATGACTT
TG-3’ (reverse, SEQ ID NO: 28)
(xxii) Primer set for LMα5 (Ile²⁷¹⁶-Ala³³¹⁰) amplification

5'-CAAAGTCATAGAAAAAGTACAGCATATTGGCCGCGTGCGAGAGCTCA
TTG-3' (forward, SEQ ID NO: 29)

   5'-CCCTTGATGGCTGGCAACTAGAAGGCACAGTCGAGGC-3' (reverse, SEQ ID NO: 30)
(xxiii) Primer set for amplification of mouse Ig-κ chain V-J2-C signal peptide/FBGγ (Tyr²⁷-Ser¹³²)
   5' -CGGGACTTTCCAAAATGTCGTAACAACTCCGCCCCATTGAC-3' (forward, SEQ ID NO: 27) 
5'-GAGTGAACGGACTTCTCCTTCCAGTCTTGCTAAACTTGAGTCATGTG
TTAAAATCGATGC-3' (reverse, SEQ ID NO: 31)
(xxiv) Primer set for LMβ1 (Leu¹⁷⁶¹-Leu¹⁷⁸⁶) amplification

5’-GCATCGATTTTAACACATGACTCAAGTTTAGCAAGACTGGAAGGAGA
AGTCCGTTCACTC-3’ (forward, SEQ ID NO: 32)

   5'-CCCTTGATGGCTGGCAACTAGAAGGCACAGTCGAGGC-3' (reverse, SEQ ID NO: 30)
(xxv) Primer set for amplification of mouse Ig-κ chain V-J2-C signal peptide/FBGβ (Gln³¹-Asn¹⁹⁴)
   5' -CGGGACTTTCCAAAATGTCGTAACAACTCCGCCCCATTGAC-3' (forward, SEQ ID NO: 27) 
5'-GCGAATGTCCTTCATGATCTCCTCGATGTTAGTTGGGATATTGCTATTC
ACAGTCTCATC-3' (reverse, SEQ ID NO: 33)
(xxvi) Primer set for LMγ1 (Ile¹⁵⁷⁹-Pro¹⁶⁰⁹) amplification

5’-GATGAGACTGTGAATAGCAATATCCCAACTAACATCGAGGAGATCATG
AAGGACATTCGC-3’ (forward, SEQ ID NO: 34)

   5'-CCCTTGATGGCTGGCAACTAGAAGGCACAGTCGAGGC-3' (reverse, SEQ ID NO: 30)

The six DNA fragments thus obtained were subjected to extension PCR using the primer sets shown below to amplify three different ligated DNA fragments. The amplified DNA fragments were separately digested with the restriction enzymes NheI and NotI and then ligated into the NheI-NotI site of pcDNA3.4+MCS to construct expression vectors for Chimera-α5, Chimera-β1, and Chimera-γ1, i.e., expression vectors for Combination-1.
(xxvii) Primer set for ligation and amplification of each chain
5'-CGGGACTTTCCAAAATGTCGTAACAACTCCGCCCCATTGAC-3' (forward, SEQ ID NO: 27)
5'-CCCTTGATGGCTGGCAACTAGAAGGCACAGTCGAGGC-3' (reverse, SEQ ID NO: 30)

### Construction of expression vectors for Combination-2

Expression vectors for Chimera-α5 consisting of mouse Ig-κ chain V-J2-C signal peptide/FBGγ (Tyr²⁷-Ser¹³²)/LMα5 (Ile²⁷¹⁶-Ala³³¹⁰)/10×His, Chimera-β1 consisting of mouse Ig-κ chain V-J2-C signal peptide/FBGβ (Gln³¹-Asn¹⁹⁴)/LMβ1 (Leu¹⁷⁶¹-Leu¹⁷⁸⁶), and Chimera-γ1 consisting of mouse Ig-κ chain V-J2-C signal peptide/FBGα (Ala²⁰-His¹⁵¹)/LMγ1 (Ile¹⁵⁷⁹-Pro¹⁶⁰⁹) were constructed as follows. First, the expression vectors for mouse Ig-κ chain V-J2-C signal peptide/LMα5 (Ile²⁷¹⁶-Ala³³¹⁰)/10×His, mouse Ig-κ chain V-J2-C signal peptide/LMβ1 (Leu¹⁷⁶¹-Leu¹⁷⁸⁶), mouse Ig-κ chain V-J2-C signal peptide/LMγ1 (Ile¹⁵⁷⁹-Pro¹⁶⁰⁹), mouse Ig-κ chain V-J2-C signal peptide/FBGβ (Gln³¹-Asn¹⁹⁴)/LMα5 (Ile²⁷¹⁶-Ala³³¹⁰)/10×His, mouse Ig-κ chain V-J2-C signal peptide/FBGα (Ala²⁵-His¹⁵¹)/LMβ1 (Leu¹⁷⁶¹-Leu¹⁷⁸⁶), and mouse Ig-κ chain V-J2-C signal peptide/FBGγ (Tyr²⁷-Ser¹³²)/LMγ1 (Ile¹⁵⁷⁹-Pro¹⁶⁰⁹) described in Example 1 were used as templates to amplify DNA fragments encoding mouse Ig-κ chain V-J2-C signal peptide/FBGγ (Tyr²⁷-Ser¹³²), LMα5 (Ile²⁷¹⁶-Ala³³¹⁰)/10×His, mouse Ig-κ chain V-J2-C signal peptide/FBGβ (Gln³¹-Asn¹⁹⁴), LMβ1 (Leu¹⁷⁶¹-Leu¹⁷⁸⁶), mouse Ig-κ chain V-J2-C signal peptide/FBGα (Ala²⁰-His¹⁵¹), and LMγ1 (Ile¹⁵⁷⁹-Pro¹⁶⁰⁹) by PCR using their respective primer sets shown below. The reverse primer of each primer set has a sequence used for extension PCR at the 5' end.
(xxviii) Primer set for amplification of mouse Ig-κ chain V-J2-C signal peptide/FBGγ (Tyr²⁷-Ser¹³²)
   5'-CGGGACTTTCCAAAATGTCGTAACAACTCCGCCCCATTGAC-3' (forward, SEQ ID NO: 27)
   5'-GAGCTCTCGCACGCGGCCAATACTTGAGTCATGTGTTAAAATCG-3' (reverse, SEQ ID NO: 35)
(xxix) Primer set for LMα5 (Ile²⁷¹⁶-Ala³³¹⁰)/10×His amplification
   5'-CGATTTTAACACATGACTCAAGTATTGGCCGCGTGCGAGAGCTC-3' (forward, SEQ ID NO: 36)
   5'-CCCTTGATGGCTGGCAACTAGAAGGCACAGTCGAGGC-3' (reverse, SEQ ID NO: 30)
(xxx) Primer set for amplification of mouse Ig-κ chain V-J2-C signal peptide/FBGβ (Gln³¹-Asn¹⁹⁴)
   5' -CGGGACTTTCCAAAATGTCGTAACAACTCCGCCCCATTGAC-3' (forward, SEQ ID NO: 27) 
5’-GGAGTGAACGGACTTCTCCTTCCAGTCTTGCTAAGTTAGTTGGGATAT
TGCTATTCACAGTCTCATC-3’ (reverse, SEQ ID NO: 37)
(xxxi) Primer set for LMβ1 (Leu¹⁷⁶¹-Leu¹⁷⁸⁶) amplification

5’-GATGAGACTGTGAATAGCAATATCCCAACTAACTTAGCAAGACTGGA
AGGAGAAGTCCGTTCACTCC-3’ (forward, SEQ ID NO: 38)

   5'-CCCTTGATGGCTGGCAACTAGAAGGCACAGTCGAGGC-3' (reverse, SEQ ID NO: 30)
(xxxii) Primer set for amplification of mouse Ig-κ chain V-J2-C signal peptide/FBGα (Ala²⁰-His¹⁵¹)
   5'-CGGGACTTTCCAAAATGTCGTAACAACTCCGCCCCATTGAC-3' (forward, SEQ ID NO: 27) 
5’-GCGAATGTCCTTCATGATCTCCTCGATATGCTGTACTTTTTCTATGACT
TTGCGCTTC-3’ (reverse, SEQ ID NO: 39)
(xxxiii) Primer set for LMγ1 (Ile¹⁵⁷⁹-Pro¹⁶⁰⁹) amplification

5’-GAAGCGCAAAGTCATAGAAAAAGTACAGCATATCGAGGAGATCATGA
AGGACATTCGC-3’ (forward, SEQ ID NO: 40)

   5'-CCCTTGATGGCTGGCAACTAGAAGGCACAGTCGAGGC-3' (reverse, SEQ ID NO: 30)

### (2) Confirmation of expression of chimeric proteins having different combinations of human fibrinogen Aα, Bβ, and y chains and human laminin α5, β1, and γ1 chains

The expression vectors for Combination-1, Combination-2, or Combination-3 were transfected into 293-F cells. Seventy-two hours after the start of culture, the culture media were collected. Each culture supernatant was analyzed for the expression of the chimeric protein of interest by SDS-PAGE under non-reducing conditions. A mixture of 5 µL of the culture supernatant and 1.3 µL of 5× SDS sample treatment buffer was treated at 95°C for 5 minutes. This mixture was applied on a 5-20% gradient polyacrylamide gel (ATTO, #2331830) and electrophoresed at 15 mA for 115 minutes. After electrophoresis, the separated proteins were transferred to a polyvinylidene difluoride membrane. The chimeric proteins transferred on the membrane were detected using an antibody (Penta-His HRP conjugate; QIAGEN, #34460) against the 10×His tag fused to the C-terminus of Chimera-aS in each combination.

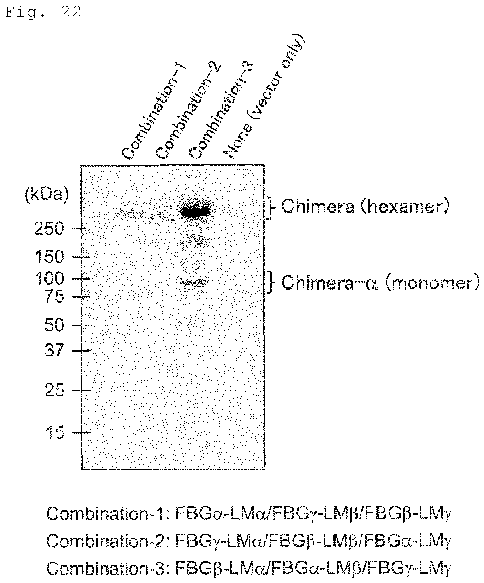

The results are shown in Fig. 22. Among the three combinations of the fibrinogen and laminin constituent subunit chains, the chimeric protein expression was the highest in Combination-3, and a major band was detected at the position corresponding to the expected hexameric molecular weight. The expression level observed in Combination-1 or Combination-2 was lower than that observed in Combination-3, but a major band was detected at the position corresponding to the expected hexameric molecular weight. These results indicate that the combination of the constituent chains of the chimeric protein (fibrinogen Aα, Bβ, and γ chains and laminin α, β, and γ chains) is not limited to any particular combination, as long as the trimeric structures of fibrinogen and laminin are retained.

The present invention is not limited to the particular embodiments and examples described above, and various modifications can be made within the scope of the appended claims. Other embodiments provided by suitably combining technical means disclosed in separate embodiments of the present invention are also within the technical scope of the present invention. All the academic publications and patent documents cited in the description are incorporated herein by reference.

## Claims

1. A chimeric protein comprising a fibrinogen fragment capable of binding to fibrinogen upon thrombin treatment and a laminin fragment having integrin-binding activity.

2. The chimeric protein according to claim 1, further comprising a protein having growth factor-binding activity.

3. The chimeric protein according to claim 2, wherein the protein having growth factor-binding activity is a heparan sulfate proteoglycan.

4. A gel formed of fibrin and a molecule generated by thrombin treatment of the chimeric protein according to any one of claims 1 to 3.

5. A method for producing the gel according to claim 4, comprising the step of preparing a mixture of the chimeric protein according to any one of claims 1 to 3, fibrinogen, and thrombin.

6. The method according to claim 5, wherein the molar ratio of the chimeric protein to the fibrinogen in the mixture is 1:5 to 1:20,000.

7. A composition for gel preparation comprising the chimeric protein according to any one of claims 1 to 3 and fibrinogen.

8. A kit for preparing the gel according to claim 4, the kit comprising the chimeric protein according to any one of claims 1 to 3, fibrinogen, and thrombin.

9. A method for three-dimensional culture of cells or tissue fragments using the gel according to claim 4.

10. A method for producing cells or organoids for transplantation medicine using the method according to claim 9.
